# EUROPÄISCHE PATENTSCHRIFT

(11) **EP 3 497 081 B1**
(45) Veröffentlichungstag und Bekanntmachung des Hinweises auf die Patenterteilung: **16.09.2020**
(21) Anmeldenummer: 17754611.6
(22) Anmeldetag: 10.08.2017
(51) Int. Cl.: C07D 211/58, C07D 211/94

(54) **VERFAHREN ZUR HERSTELLUNG VON 4-AMMONIUM-2,2,6,6,-TETRAALKYLPIPERIDINYLSALZEN**
PROCESS FOR PREPARING 4-AMMONIUM-2,2,6,6-TETRAALKYLPIPERIDINYL SALTS
PROCÉDÉ POUR PRÉPARER DES SELS DE 4-AMMONIUM-2,2,6,6-TÉTRAALKYLPIPÉRIDINYLE

(30) Priorität: 12.08.2016 DE 102016009904
(43) Veröffentlichungstag der Anmeldung: 19.06.2019
(73) Patentinhaber: JenaBatteries GmbH, 07745 Jena (DE)
(72) Erfinder: SCHUBERT, Ulrich, Sigmar, 07743 Jena (DE); JANOSCHKA, Tobias, 07743 Jena (DE); MARTIN, Norbert, 08297 Zwönitz (DE); HAGER, Martin, 07774 Dornburg Camburg (DE)
(74) Vertreter: Ackermann, Joachim
(86) Internationale Anmeldenummer: PCT/EP2017/000968
(87) Internationale Veröffentlichungsnummer: WO 2018/028830

(56) Entgegenhaltungen:
- EP-A1- 3 048 097
- EP-A2- 0 302 020
- CHARLES FRANCAVILLA ET AL: "Novel-chloroheterocyclic antimicrobials", BIOORGANIC & MEDICINAL CHEMISTRY LETTERS, PERGAMON, AMSTERDAM, NL, Bd. 21, Nr. 10, 10. März 2011 (2011-03-10) , Seiten 3029-3033, XP028208773, ISSN: 0960-894X, DOI: 10.1016/J.BMCL.2011.03.035 [gefunden am 2011-03-16] in der Anmeldung erwähnt
- BARNEY L. BALES ET AL: "Electron Paramagnetic Resonance Line Shifts and Line Shape Changes Due to Spin Exchange of Nitroxide Free Radicals in Liquids. 7. Singly Charged Surfactant Nitroxide", JOURNAL OF PHYSICAL CHEMISTRY. A, MOLECULES, SPECTROSCOPY,KINETICS, ENVIRONMENT AND GENERAL THEORY, Bd. 113, Nr. 33, 20. August 2009 (2009-08-20), Seiten 9295-9303, XP055407919, US ISSN: 1089-5639, DOI: 10.1021/jp905335r
- BIRANDRA K. SINHA ET AL: "Synthesis and biological activity of spin-labeled analogs of biotin, hexamethonium, decamethonium, dichlorisoproterenol, and propranolol", JOURNAL OF MEDICINAL CHEMISTRY, Bd. 18, Nr. 7, 1. Juli 1975 (1975-07-01), Seiten 669-673, XP055407895, ISSN: 0022-2623, DOI: 10.1021/jm00241a005

## Beschreibung

Die Erfindung betrifft ein einfaches Verfahren zur Herstellung von 4-Ammonium-2,2,6,6-tetraalkylpiperidinylsalzen. Derartige Verbindungen eignen sich als redoxaktive Materialien und können in unterschiedlichen elektrochemischen Zellen eingesetzt werden. Diese weisen Anoden- und Kathodenräume auf, in denen sich redoxaktive Materialien befinden. An der Kathode findet beim Entladen der elektrochemischen Zelle die Reduktion der redoxaktiven Komponente statt und an der Anode findet beim Entladen der elektrochemischen Komponente die Oxidation der redoxaktiven Komponente statt.

Vorzugsweise werden 4-Ammonium-2,2,6,6-tetraalkylpiperidinylsalze im Kathodenraum von Redox-Fluss-Batterien eingesetzt. Der synthetisch einfache Zugang zu solchen Verbindungen ist wünschenswert um die Wirtschaftlichkeit von elektrochemischen Zellen zu verbessern.

In der Literatur sind verschiedene Strategien zur Herstellung von 4-Ammonium-2,2,6-6-tetraalkylpiperidinylsalzen und ähnlicher Strukturen bekannt. Diese gehen entweder von 4-Oxo-2,2,6,6-tetraalkylpiperidinylverbindungen aus oder von deren Oxidationsprodukt. Die erste Syntheseroute setzt teure Reduktionsmittel und eine auf-wendige Reinigung voraus. Die zweite Syntheseroute geht mit dem Problem einher, dass im Reaktionsschritt der reduktiven Aminierung selektive Reduktionsmittel genutzt werden müssen, die nur das Amin reduzieren, nicht aber das NO-Radikal. Diese sind teuer und führen in einer Nebenreaktion zu einem Hydroxylammonium-Produkt, dass abgetrennt werden muss.

So beschreiben Francavilla et al. in Bioorganic and Medicinal Chemistry Letters, 2011, Vol. 21, # 10 S. 3029 - 3033 die Umsetzung von 4-Dimethylamino-2,2,6,6-tetramethylpiperidin mit Methyliodid zum entsprechenden 4-Trimethylammoniumiodid.

Kwan et al. beschreiben in J. Am. Chem. Soc., 1978, 100 (15), pp. 4783-6 die Umsetzung von 4-(N,N-Dimethylamino)-2,2,6,6-tetramethylpiperidin mit n-Hexadecylbormid zum entsprechenden 4-(N,N-Dimethyl-N-hexadecyl)-ammoniumbromid.

Von Nakatsuji et al. in Journal of Materials Chemistry 1999, Vol. 9, S. 1747 - 1754 oder von Aonuma et al. in Journal of Materials Chemistry, 2001, Vol. 11, # 2, S. 337 - 345 wird folgende Reaktionssequenz beschrieben: reduktive Aminierung von 4-Oxo-2,2,6,6-tetra-methylpiperidin-1-yloxyl mit Dimethylammoniumhydrochlorid in Gegenwart von Natriumcyanoborohydrid zum N,N-Dimethyl-4-amino-2,2,6,6-tetramethylpiperidin-1-yloxyl gefolgt von der Methylierung mit Methyliodid zum N, N, N-Trimethyl-4-ammonium-2,2,6,6-tetramethylpiperidin-1-yloxyliodid.

Strehmel et al. offenbaren in Tetrahedron Letters, 2008, Vol. 49, S. 3264-3267 die Synthese von 4-Trimethylammonium-2,2,6,6-tetramethylpiperidin-1-yloxyl. Dazu wurden 4-Amino-2,2,6,6-tetraalkylpiperidinyloxyradikale mit Methyliodid am 4-Aminstickstoff zum Trimethyl-ammoniumderivat alkyliert und das erhaltene 4-Trimethylammonium-iodid wurde durch Umsetzung mit Silbersalzen, bei der Silberiodid ausgefällt wurde, in Trimethylammoniumsalze mit anderen Anionen überführt. 4-Amino-2,2,6,6-tetramethylpiperidin-1-yloxyl selbst ist ein teurer Ausgangsstoff, der wiederum in mehreren Schritten aus 4-Oxo-2,2,6,6-tetramethylpiperidin hergestellt werden muss.

Raukman et al. beschreiben in Organic Preparations and Procedures International, 1977, Vol. 9, 2, S. 53-56 die Verwendung von Natriumcyanoborohydriden zur Herstellung von biradikalischen Nitroxiden. Diese Verbindungen werden ausgehend von 4-Oxo-2,2,6,6-tetramethylpiperidinyloxyradikalen hergestellt, indem diese Radikale mit Methylamin und Natriumcyanoborohydrid reduktiv aminiert werden.

Ferner wird die Umsetzung dieses Produktes mit Methyliodid zum 4-Ammoniumiodid offenbart.

Merbouh et al. beschreiben in Organic Preparations and Procedures International, 2004, Vol. 36, # 1, S. 1-31 verschiedene Methoden zur Herstellung von Tetramethylpiperidin-1-oxoammoniumsalzen. Auf S. 23 und 24 werden Synthesen von 4-Amino-2,2,6,6-tetramethylpiperidin-N-oxid beschrieben. Diese gehen von 4-Oxo-2,2,6,6-tetramethyl-piperidin-N-oxid oder von 4-Acetamido-2,2,6,6-tetramethylpiperidin-N-oxid aus.

Auch die reduktive Aminierung von Cyclohexanon-Derivaten ist bekannt. So wird in Anales de Quimica, 1998, Vol. 94, # 1, S. 36 - 45 und in Collection of Czeceslovak Chemical Communications 1987, Vol. 52, # 1, S. 185-192 die elektrochemische Umsetzung von 4-Oxo-2,2,6,6-tetraalkylpiperidin mit Hydroxylamin oder Hydrazin zu 4-Amino-2,2,6,6-tetraalkylpiperidin beschrieben.

Darüber hinaus ist auch die Umsetzung von Cyclohexanon zu Cyclohexylamin mit Wasserstoff am Metallkontakt bekannt.

Aus Bulletin de la Societe Chimique de France, 1886, Vol. 2, # 45, S. 501 ist schließlich bekannt, dass Dimethylamin durch Chlormethan methyliert werden kann.

Auch sind weitere Umsetzungen von tertiären Aminogruppen mit Chlormethan zu quaternären Ammoniumchloriden beschrieben worden.

Barney et al. beschreiben in J. Phys. Chem. A 2009, 113, 9295-9303 EPR-Untersuchungen an 4-[N, N-Dimethyl-N-(n-dodecyl)ammonium]-2,2,6,6-tetramethylpiperidinyl-N-oxy-bromid-d₁₆. In diesem Zusammenhang wird die Herstellung von 4-n-Dodecyldimethylammonium-3,3,5,5-tetradeuterio-2,2,6,6-tetrakis(trideuteriomethyl)-piperidinbromid offenbart. Die Synthese erfolgt durch Umsetzung von n-Bromdodecan mit 4-(Dimethylamino-3,3,5,5-tetradeuterio-2,2,6,6-tetrakis(trideuteriomethyl)-piperidin.

Biandra et al. beschreiben in Journal of Medical Chemistry, 1975, Vol. 18, No. 7, 669-673 die Synthese von Biotin-Analogen. In diesem Zusammenhang wird die Herstellung von 4,4'-[1,6-Hexandiyl-bis-(dimethylamino)]-bis-[2,2,6,6,-tetramethylpiperidin]-dibromid und von 4,4'-[1,10-Decandiyl-bis-(dimethylamino)]-bis-[2,2,6,6,-tetramethylpiperidin]-dibromid offenbart. Die Synthese erfolgt durch die Umsetzung von 4,4-Dimethylamino-2,2,6,6-tetramethylpiperidin mit 1,6-Dibromhexan bzw. mit 1,10-Dibromdecan.

Es wurde jetzt ein einfach durchzuführendes Verfahren zur Herstellung von 4-Ammonium-2,2,6,6-tetraalkylpiperidinylsalzen gefunden, das von 4-Oxo-2,2,6,6-tetraalkylpiperidinylverbindungen und nicht von deren schwieriger zu handhabenden Oxidationsprodukten ausgeht und das ohne teure Reduktionsmittel auskommt und keine aufwendigen Reinigungsschritte benötigt. Außerdem gestattet das erfindungsgemäße Verfahren die Herstellung von 4-Ammonium-2,2,6,6-tetraalkylpiperidinyl-salzen in hoher Ausbeute und-Reinheit. Es ermöglicht darüber hinaus den direkten Zugang zu den Chloriden oder Sulfaten der 4-Ammonium-2,2,6,6-tetraalkylpiperidin-ylsalze, ohne dass ein aufwendiger Schritt des Ionenaustausches wie im Beispiel der Methylierung mit Methyliodid nötig wird. Diese Chloride oder Sulfate können zur Herstellung redoxaktiver Verbindungen eingesetzt werden, die in Batterien, vorzugsweise in Redox-Flow-Batterien eingesetzt werden können.

Die vorliegende Erfindung betrifft ein Verfahren zur Herstellung von 2,2,6,6-Tetraalkylpiperidin-4-ammoniumsalzen der Formel I oder der Formel II 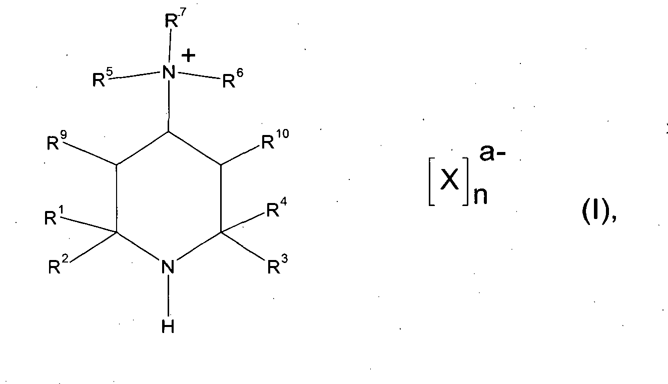 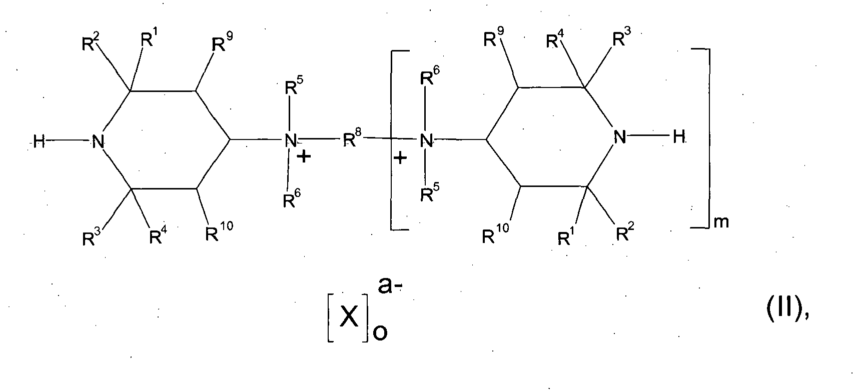
worin R¹, R², R³ und R⁴ C₁-C₆-Alkyl sind,
R⁹ und R¹⁰ unabhängig voneinander Wasserstoff oder C₁-C₆-Alkyl bedeuten,
R⁵ und R⁶ unabhängig voneinander Wasserstoff, Alkyl, Cycloalkyl, Aryl, Aralkyl oder Heterocyclyl bedeuten, die gegebenenfalls mit ein oder mehreren Resten ausgewählt aus der Gruppe bestehend aus Alkyl, Alkoxy, Hydroxy, Carboxyl, Carboxylester, Carboxylamid, Sulfonat, Sulfonsäureester, Sulfonamid oder Halogen substituiert sind, oder Alkyl, das in der Alkylgruppe durch ein oder mehrere, nicht direkt zueinander benachbarte zweiwertige Reste ausgewählt aus der Gruppe bestehend aus Sauerstoff oder Imino unterbrochen ist, oder in denen R⁵ und R⁶ zusammen eine Alkylengruppe bilden,
R⁷ Alkyl bedeutet, das gegebenenfalls mit ein oder mehreren Resten ausgewählt aus der Gruppe bestehend aus Alkyl, Alkoxy, Hydroxy, Nitro, Carboxyl, Carboxylester, Carboxylamid, Sulfonat, Sulfonsäureester, Sulfonamid oder Halogen substituiert ist, oder Alkyl, das in der Alkylgruppe durch ein oder mehrere, nicht direkt zueinander benachbarte zweiwertige Reste ausgewählt aus der Gruppe bestehend aus Sauerstoff oder Imino unterbrochen ist,
R⁸ eine zwei- bis sechswertige organische Brückengruppe ist, die gegebenen-falls mit ein oder mehreren Resten ausgewählt aus der Gruppe bestehend aus Alkyl, Alkoxy, Hydroxy, Nitro, Carboxyl, Carboxylester, Carboxylamid, Sulfonat, Sulfonsäureester, Sulfonamid oder Halogen substituiert ist, oder Alkylen, das in der Alkylengruppe durch ein oder mehrere, nicht direkt zueinander benachbarte zweiwertige Reste ausgewählt aus der Gruppe bestehend aus Sauerstoff oder Imino unterbrochen ist,
a eine ganze Zahl von 1 bis 10.000 bedeutet,
m eine ganze Zahl von 1 bis 5 bedeutet,
n eine Zahl mit dem Wert 1/a bedeutet,
o eine Zahl mit dem Wert (1 + m) / a bedeutet, und
X ein organisches Anion, ein anorganisches Anion oder eine Mischung dieser Anionen ist, vorzugsweise ein Chloridanion oder ein Sulfatanion, wobei das Verfahren die folgenden Maßnahmen umfasst:
   a) Vorlage einer Lösung von 4-Oxo-alkylpiperidin der Formel VI 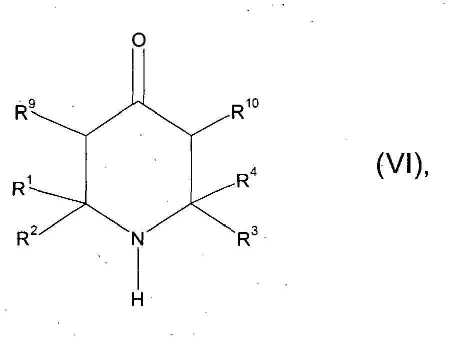 wobei R¹, R², R³, R⁴, R⁹ und R¹⁰ die oben definierte Bedeutung besitzen, und einem Amin der Formel HNR⁵R⁶, wobei R⁵ und R⁶ die oben definierte Bedeutung besitzen, in einem Lösungsmittel ausgewählt aus der Gruppe der Alkohole, Ether, Nitrile, halogenierten Kohlenwasserstoffe, aromatischen Kohlenwasserstoffe, aliphatischen Kohlenwasserstoffe oder Gemischen davon,
   b) reduktive Aminierung der 4-Oxo-alkylpiperidinylverbindung der Formel VI mittels Wasserstoff, Alkalimetallen, Magnesium, Aluminium, Zink, Alkalialuminiumhydriden, Alkaliborhydriden, Alkalihydriden, Erdalkalihydriden, Alkalisulfiten, Alkalidithioniten, Alkalithiosulfaten oder Hydrazin in Gegenwart eines Hydrierkatalysators zu einer 4-Amino-alkylpiperidinylverbindung der Formel VII 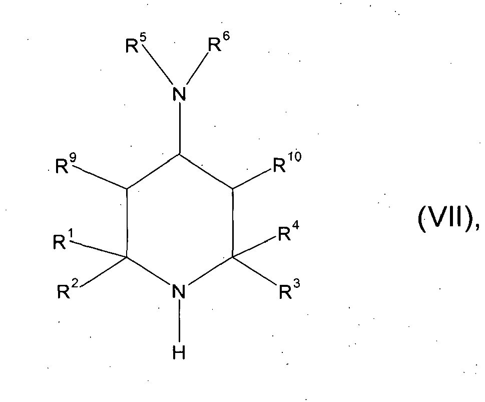 wobei R¹, R², R³, R⁴, R⁵, R⁶, R⁹ und R¹⁰ die oben definierte Bedeutung besitzen,
   c) Abtrennung des Reaktionsproduktes der Formel VII aus Schritt b) vom Hydrierkatalysator und Lösungsmittel,
   d) Auflösen des in Schritt c) abgetrennten Reaktionsproduktes der Formel VII in einem oder mehreren aprotischen organischen Lösungsmittel und Zuführen von einem organischen Chlorid der Formel R⁷-Cl oder der Formel Cl-R⁸-(Cl)ₘ oder von einem organischen Sulfat der Formel R⁷-O-SO₂-O-R⁷, worin R⁷, m und R⁸ die obigen Definitionen aufweisen,
   e) Umsetzen des Reaktionsgemisches aus Schritt d) bei einem Druck von 1 bis 500 bar und einer Temperatur von 0 bis 200°C, gegebenenfalls in Anwesenheit einer Base
   f) gegebenenfalls nach Schritt e) oder gegebenenfalls nach Schritt g) Austausch des Chloridanions oder des Sulfatanions durch ein anderes Anion, und
   g) gegebenenfalls Abtrennen des Reaktionsproduktes aus dem Reaktionsgemisch
   oder, wobei das Verfahren die folgenden Maßnahmen umfasst:
   a') Vorlage einer Lösung eines Imins der Formel V 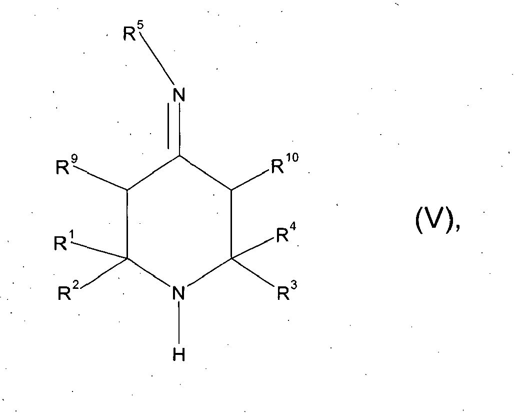 wobei R¹, R², R³, R⁴, R⁵, R⁹ und R¹⁰ die oben definierte Bedeutung besitzen, in einem Lösungsmittel ausgewählt aus der Gruppe der Alkohole, Ether, Nitrile, halogenierten Kohlenwasserstoffe, aromatischen Kohlenwasserstoffe, aliphatischen Kohlenwasserstoffe oder Gemischen davon,
   b) Hydrierung des Imins der Formel V mittels Wasserstoff, Alkalimetallen, Magnesium, Aluminium, Zink, Alkalialuminiumhydriden, Alkaliborhydriden, Alkalihydriden, Erdalkalihydriden, Alkalisulfiten, Alkalidithioniten, Alkalithiosulfaten oder Hydrazin in Gegenwart eines Hydrierkatalysators zu einer 4-Amino-alkylpiperidinylverbindung der Formel VII 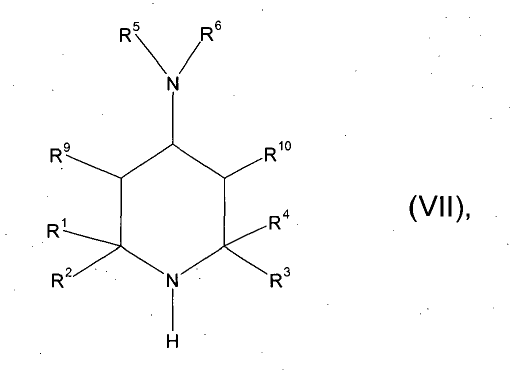 wobei R¹, R², R³, R⁴, R⁵, R⁶, R⁹ und R¹⁰ die oben definierte Bedeutung besitzen,
   c) Abtrennung des Reaktionsproduktes der Formel VII aus Schritt b) vom Hydrierkatalysator und Lösungsmittel,
   d) Auflösen des in Schritt c) abgetrennten Reaktionsproduktes der Formel VII in einem oder mehreren aprotischen organischen Lösungsmittel und Zuführen von einem organischen Chlorid der Formel R⁷-Cl oder der Formel Cl-R⁸-(Cl)ₘ oder von einem organischen Sulfat der Formel R⁷-O-SO₂-O-R⁷, worin R⁷, m und R⁸ die obigen Definitionen aufweisen,
   e) Umsetzen des Reaktionsgemisches aus Schritt d) bei einem Druck von 1 bis 500 bar und einer Temperatur von 0 bis 200°C, gegebenenfalls in Anwesenheit einer Base
   f) gegebenenfalls nach Schritt e) oder gegebenenfalls nach Schritt g) Austausch des Chloridanions oder des Sulfatanions durch ein anderes Anion, und
   g) gegebenenfalls Abtrennen des Reaktionsproduktes aus dem Reaktionsgemisch
   oder, wobei das Verfahren die folgenden Maßnahmen umfasst:
   a") Vorlage von 4-Amino-alkylpiperidin der Formel VII 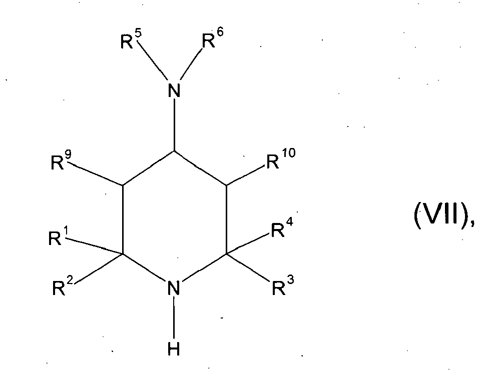 wobei R¹, R², R³, R⁴, R⁵, R⁶, R⁹ und R¹⁰ die oben definierte Bedeutung besitzen, in einem oder mehreren aprotischen organischen Lösungsmittel,
   d') Zuführen von einem organischen Chlorid der Formel R⁷-Cl oder der Formel Cl-R⁸-(Cl)ₘ oder von einem organischen Sulfat der Formel R⁷-O-SO₂-O-R⁷, worin R⁷, m und R⁸ die obigen Definitionen aufweisen, zur Lösung aus Schritt a),
   e) Umsetzen des Reaktionsgemisches aus Schritt d') bei einem Druck von 1 bis 500 bar und einer Temperatur von 0 bis 200°C, gegebenenfalls in Anwesenheit einer Base
   f) gegebenenfalls nach Schritt e) oder gegebenenfalls nach Schritt g) Austausch des Chloridanions oder des Sulfatanions durch ein anderes Anion, und
   g) gegebenenfalls Abtrennen des Reaktionsproduktes aus dem Reaktionsgemisch.

Überraschend an dem erfindungsgemäßen Verfahren ist, dass die Reaktion in Schritt e) nur an 4-Aminogruppen und nicht an der sekundären 1-Aminogruppe erfolgt.

Die Reste R¹ bis R⁴ sind verzweigte oder unverzweigte Alkylgruppen mit ein bis zu sechs Kohlenstoffatomen, bevorzugt mit ein bis vier Kohlenstoffatomen, besonders bevorzugt mit einem Kohlenstoffatom. Beispiele für Alkylgruppen sind: Methyl, Ethyl, n- Propyl, Isopropyl, *n*-Butyl, *sec*-Butyl, *tert.-Butyl,* Pentyl oder n-Hexyl. Besonders bevorzugt werden Ethyl und insbesondere Methyl.

Die Reste R⁹ und R¹⁰ sind Wasserstoff oder verzweigte oder unverzweigte Alkylgruppen mit ein bis zu sechs Kohlenstoffatomen. Beispiele dafür sind oben bei der Beschreibung der Reste R¹ bis R⁴ aufgeführt.

Bevorzugt ist R⁹ Wasserstoff und R¹⁰ ist Alkyl mit ein bis sechs Kohlenstoffatomen.

Ganz besonders bevorzugt sind R⁹ und R¹⁰ Wasserstoff.

Die Ausgangsprodukte der Formeln V, VI oder VII sind zum Teil kommerziell verfügbar oder lassen sich nach Standardreaktionen der organischen Chemie herstellen.

Bei dem in Schritt a) eingesetzten Amin handelt es sich um eine Verbindung der Formel HNR⁵R⁶. Beispiele dafür sind Ammoniak, primäre oder sekundäre Amine, in denen R⁵ Wasserstoff und R⁶ Alkyl bedeuten oder in denen R⁵ und R⁶ Alkyl bedeuten.

Bevorzugt eingesetzt werden Amine der Formel HNR⁵R⁶, in denen R⁵ und R⁶ Alkyl, vorzugsweise C₁-C₆-Alkyl bedeuten oder in denen R⁵ und R⁶ zusammen eine Alkylengruppe bilden.

Besonders bevorzugte Beispiele für Amine der Formel HNR⁵R⁶ sind Dimethylamin, Diethylamin, Di-N-propylamin, Di-N-butylamin, N-Propyl-N-butylamin, Methylethylamin, Piperidin, Morpholin, Piperazin, N,N'-Dimethylethylendiamin, N,N',N"-Trimethyldiethylentriamin oder Pyrrolidin.

Bevorzugt in Schritt a) eingesetzte Amine der Formel HNR⁵R⁶ sind sekundäre Amine.

Das molare Verhältnis von 4-Oxo-alkylpiperidin der Formel VI zu Amin der Formel HNR⁵R⁶ in Schritt a) beträgt üblicherweise 1 : 6 bis 1 : 1, insbesondere 1 : 4 bis 1: 1 und ganz besonders bevorzugt 1 : 3 bis 1 : 1.

Bei dem in Schritten a) - c) oder a') - c) verwendeten Lösungsmittel handelt es sich um einen Alkohol, Ether, Nitril, halogenierten Kohlenwasserstoff, aromatischen Kohlenwasserstoff oder aliphatischen Kohlenwasserstoff. Es können auch Gemische von zwei oder mehreren dieser Lösungsmittel verwendet werden. Bevorzugt werden Alkohole eingesetzt.

Beispiele für Alkohole sind aliphatische Alkohole, ganz besonders bevorzugt aliphatische Alkohole mit ein bis sechs Kohlenstoffatomen und insbesondere Ethanol oder Methanol. Es können auch Gemische von Alkoholen eingesetzt werden.

Beispiele für Ether sind Diethylether und Diisopropylether.

Beispiele für Nitrile sind Acetonitril.

Beispiele für halogenierte Kohlenwasserstoffe sind chlorierte und/oder fluorierte aliphatische Kohlenwasserstoffe, wie Dichlormethan, Chloroform, Tetrachlorkohlenstoff, Dichlorethan oder Chlorfluorethan.

Beispiele für aromatische Kohlenwasserstoffe sind Benzol, Toluol oder Xylol.

Beispiele für aliphatische Kohlenwasserstoffe sind Hexan, Octan oder Decan sowie Petrolether oder Petroleum.

Bei dem in Schritt d) oder d') eingesetzten Chlorid mit einem Chloratom handelt es sich um eine Verbindung der Formel R⁷-Cl. Bevorzugt werden Alkylchloride. Beispiele dafür sind Alkylchloride, deren Alkylgruppe ein bis sechs Kohlenstoffatome aufweist. Bevorzugt befindet sich das Chloratom am endständigen Kohlenstoffatom. Bei dem in Schritt d) oder d') eingesetzten Chlorid mit zwei bis sechs Chloratomen handelt es sich um eine Verbindung der Formel Cl-R⁸-(Cl)ₘ. Bevorzugt werden Alkyldi-, Alkyltri-, Alkylquater-, Alkylpenta- und Alkylhexachloride. Beispiele dafür sind Alkyldichloride oder Alkyltrichloride, deren Alkylengruppen oder Alkyltriylgruppen ein bis sechs Kohlenstoffatome aufweisen.

Bei dem in Schritt d) oder d') eingesetzten organischen Sulfat handelt es sich um eine Verbindung der Formel R⁷-O-SO₂-O-R⁷. Bevorzugt werden Dialkylsulfate, insbesondere Dimethylsulfat.
Bevorzugt wird in Schritt d) oder d') des erfindungsgemäßen Verfahrens ein organisches Chlorid eingesetzt.

Bevorzugte Beispiele für Alkylchloride der Formel R⁷-Cl sind Methylchlorid, oder Ethylchlorid. Ganz besonders bevorzugt ist Methylchlorid.

Das molare Verhältnis von 4-Amino-alkylpiperidin der Formel VII zu Alkylchlorid der Formel R⁷-Cl in Schritt d) oder d') beträgt üblicherweise 1 : 5 bis 1 : 1, insbesondere 1 : 3 bis 1: 1 und ganz besonders bevorzugt 1 : 2 bis 1 : 1.

Das molare Verhältnis von 4-Amino-alkylpiperidin der Formel VII zu orqanischem Chlorid der Formel Cl-R⁸-(Cl)ₘ in Schritt d) oder d') beträgt üblicherweise 1 : 5 x m bis 1 : m, insbesondere 1 : 3 x m bis 1: m und ganz besonders bevorzugt 1 : 2 x m bis 1 : m, worin m die oben definierte Bedeutung besitzt.

Das molare Verhältnis von 4-Amino-alkylpiperidin der Formel VII zu organischem Sulfat der Formel R⁷-O-SO₂-O-R⁷ in Schritt d) oder d') beträgt üblicherweise 1 : 5 bis 1 : 1, insbesondere 1 : 3 bis 1: 1 und ganz besonders bevorzugt 1 : 2 bis 1 : 1.

Bedeutet einer der Reste R⁵, R⁶ und/oder R⁷ Alkyl, so kann die Alkylgruppe sowohl verzweigt als auch unverzweigt sein. Eine Alkylgruppe dieser Reste enthält typischerweise ein bis zu zwanzig Kohlenstoffatome, bevorzugt ein bis zu zehn Kohlenstoffatome. Beispiele für Alkylgruppen sind: Methyl, Ethyl, n-Propyl, Isopropyl, n-Butyl, sec-Butyl, *tert*.-Butyl, Pentyl, n-Hexyl, n-Heptyl, 2-Ethylhexyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl oder Eicosyl. Besonders bevorzugt sind Alkylgruppen mit ein bis sechs Kohlenstoffatomen.

Die Reste R⁵, R⁶ und/oder R⁷ als Alkyl können gegebenenfalls substituiert sein, beispielsweise mit Alkoxy, Hydroxy, Carboxyl, Carboxylester, Carboxylamid, Sulfonat, Sulfonsäureester, Sulfonamid oder Halogen. Der Rest R⁷ als Alkyl kann gegebenenfalls auch mit Nitro substituiert sein.

Die Reste R⁵, R⁶ und/oder R⁷ als Alkyl können gegebenenfalls durch ein oder mehrere, nicht direkt zueinander benachbarte zweiwertige Sauerstoff- oder Iminoreste unterbrochen sein. Beispiele dafür sind einwertige Reste abgeleitet von Polyethylenglykolen, von Polypropylenglykolen, von Polyethyleniminen oder von Polypropyleniminen.

Bedeutet einer der Reste R⁵ und/oder R⁶ Cycloalkyl, so ist die Cycloalkylgruppe typischerweise eine cyclische Gruppe, enthaltend fünf bis acht, vorzugsweise fünf, sechs oder sieben Ringkohlenstoffatome, die jeweils unabhängig voneinander substituiert sein können. Beispiele für Substituenten sind Alkylgruppen oder zwei Alkylgruppen, die zusammen mit den Ringkohlenstoffen, an denen sie gebunden sind, einen weiteren Ring bilden können. Beispiele für Cycloalkylgruppen sind Cyclopentyl oder Cyclohexyl. Cycloalkylgruppen können gegebenenfalls mit Alkyl, Alkoxy, Hydroxy, Carboxyl, Carboxylester, Carboxylamid, Sulfonat, Sulfonsäureester, Sulfonamid oder Halogen substituiert sein.

Bedeutet einer der Reste R⁵ und/oder R⁶ Aryl, so ist die Arylgruppe typischerweise eine cyclische aromatische Gruppe, enthaltend fünf bis vierzehn Kohlenstoffatome, die jeweils unabhängig voneinander substituiert sein können. Beispiele für Substituenten sind Alkylgruppen oder zwei Alkylgruppen, die gemeinsam mit den Ringkohlenstoffatomen, an denen sie gebunden sind, einen weiteren Ring bilden können. Beispiele für Arylgruppen sind Phenyl, Biphenyl, Anthryl oder Phenantolyl. Arylgruppen können gegebenenfalls mit Alkyl, Alkoxy, Hydroxy, Carboxyl, Carboxylester, Carboxylamid, Sulfonat, Sulfonsäureester, Sulfonamid oder Halogen substituiert sein.

Bedeutet einer der Reste R⁵ und/oder R⁶ Heterocyclyl, so kann die Heterocyclylgruppe typischerweise eine cyclische Gruppe mit vier bis zehn Ringkohlenstoffatomen und mindestens einem Ringheteroatom aufweisen, die jeweils unabhängig voneinander substituiert sein können. Beispiele für Substituenten sind Alkylgruppen, oder zwei Alkylgruppen die zusammen mit den Ringkohlenstoffen an denen sie gebunden sind einen weiteren Ring bilden können. Beispiele für Heteroatome sind Sauerstoff, Stickstoff, Phosphor, Bor, Selen oder Schwefel. Beispiele für Heterocyclylgruppen sind Furyl, Thienyl, Pyrrolyl oder Imidazolyl. Heterocyclylgruppen sind vorzugsweise aromatisch. Heterocyclylgruppen können gegebenenfalls mit Alkyl, Alkoxy, Hydroxy, Carboxyl, Carboxylester, Carboxylamid, Sulfonat, Sulfonsäureester, Sulfonamid oder Halogen substituiert sein.

Bedeutet einer der Reste R⁵ und/oder R⁶ Aralkyl, so ist die Aralkylgruppe typischerweise eine Arylgruppe, wobei Aryl zuvor bereits definiert wurde, an die kovalent eine Alkylgruppe gebunden ist. Die Aralkylgruppe kann am aromatischen Ring beispielsweise mit Alkylgruppen oder mit Halogenatomen substituiert sein. Ein Beispiel für eine Aralkylgruppe ist Benzyl. Aralkylgruppen können gegebenenfalls mit Alkyl, Alkoxy, Hydroxy, Carboxyl, Carboxylester, Carboxylamid, Sulfonat, Sulfonsäureester, Sulfonamid oder Halogen substituiert sein.

Besonders bevorzugt sind R⁵ und R⁶ unabhängig voneinander Alkyl, die gegebenenfalls mit ein oder mehreren Resten ausgewählt aus der Gruppe bestehend aus Alkyl, Alkoxy, Hydroxy, Carboxyl, Carboxylester, Carboxylamid, Sulfonat, Sulfonsäureester, Sulfonamid oder Halogen substituiert sind und/oder die gegebenenfalls in der Alkylgruppe durch ein oder mehrere, nicht direkt zueinander benachbarte zweiwertige Reste ausgewählt aus der Gruppe bestehend aus Sauerstoff oder Imino unterbrochen sind.

Besonders bevorzugt ist R⁷ Alkyl, das gegebenenfalls mit ein oder mehreren Resten ausgewählt aus der Gruppe bestehend aus Alkyl, Alkoxy, Hydroxy, Nitro, Carboxyl, Carboxylester, Carboxylamid, Sulfonat, Sulfonsäureester, Sulfonamid oder Halogen substituiert ist und/oder das gegebenenfalls in der Alkylgruppe durch ein oder mehrere, nicht direkt zueinander benachbarte zweiwertige Reste ausgewählt aus der Gruppe bestehend aus Sauerstoff oder Imino unterbrochen ist.

Ganz besonders bevorzugt bedeuten R⁵, R⁶ und R⁷ unabhängig voneinander C₁-C₆-Alkyl.

Bedeutet einer der Substituenten Halogen, so ist darunter ein kovalent gebundenes Fluor-, Chlor-, Brom- oder Iodatom zu verstehen. Bevorzugt sind Chlor oder Brom.

R⁸ ist eine zwei- bis sechswertige organische Brückengruppe. Darunter ist ein organischer Rest zu verstehen, der über zwei, drei, vier, fünf oder sechs kovalente Bindungen mit dem Rest des Moleküls verbunden ist.

Beispiele für zweiwertige organische Reste sind Alkylen, Alkylenoxy, Poly(alkylenoxy), Alkylenamino, Poly(alkylenamino), Cycloalkylen, Arylen, Aralkylen oder Heterocyclylen.

Alkylengruppen können sowohl verzweigt als auch unverzweigt sein. Eine Alkylengruppe enthält typischerweise ein bis zu zwanzig Kohlenstoffatome, bevorzugt zwei bis zu vier Kohlenstoffatome. Beispiele für Alkylengruppen sind: Methylen, Ethylen, Propylen und Butylen. Alkylengruppen können gegebenenfalls substituiert sein, beispielsweise mit Alkyl, Alkoxy, Hydroxy, Carboxyl, Carboxylester, Carboxylamid, Sulfonat, Sulfonsäureester, Sulfonamid oder Halogen.

Alkylenoxy- und Poly(alkylenoxy)gruppen können sowohl verzweigte als auch unverzweigte Alkylengruppen enthalten. Eine in einer Alkylenoxy- oder in einer Poly(alkylenoxy)gruppe auftretende Alkylengruppe enthält typischerweise zwei bis vier Kohlenstoffatome, bevorzugt zwei oder drei Kohlenstoffatome. Die Anzahl der Wiederholeinheiten in den Poly(alkylenoxy)gruppen kann in weiten Bereichen schwanken. Typische Anzahlen von Wiederholeinheiten bewegen sich im Bereich von 2 bis 50. Beispiele für Alkylenoxygruppen sind: Ethylenoxy, Propylenoxy und Butylenoxy. Beispiele für Poly(alkylenoxy)gruppen sind: Poly(ethylenoxy), Poly(propylenoxy) und Poly(butylenoxy).

Alkylenamino- und Poly(alkylenamino)gruppen können sowohl verzweigte als auch unverzweigte Alkylengruppen enthalten. Eine in einer Alkylenamino- oder in einer Poly(alkylenamino)gruppe auftretende Alkylengruppe enthält typischerweise zwei bis vier Kohlenstoffatome, bevorzugt zwei oder drei Kohlenstoffatome. Die Anzahl der Wiederholeinheiten in den Poly(alkylenamino)gruppen kann in weiten Bereichen schwanken. Typische Anzahlen von Wiederholeinheiten bewegen sich im Bereich von 2 bis 50. Beispiele für Alkylenaminogruppen sind: Ethylenamino, Propylenamino und Butylenamino. Beispiele für Poly(alkylenamino)gruppen sind: Poly(ethylenamino), Poly(propylenamino) und Poly(butylenamino).

Cycloalkylengruppen enthalten typischerweise fünf, sechs oder sieben Ringkohlenstoffatome, die jeweils unabhängig voneinander substituiert sein können. Beispiele für Substituenten sind Alkylgruppen oder zwei Alkylgruppen, die zusammen mit den Ringkohlenstoffen, an denen sie gebunden sind, einen weiteren Ring bilden können. Ein Beispiel für eine Cycloalkylengruppe ist Cyclohexylen.

Arylengruppen sind typischerweise cyclische aromatische Gruppen, die fünf bis vierzehn Kohlenstoffatome enthalten, die jeweils unabhängig voneinander substituiert sein können. Beispiele für Arylengruppen sind o-Phenylen, m-Phenylen, p-Phenyl, Naphthylengruppen oder Biphenylengruppen.

Heterocyclylgruppen sind typischerweise cyclische Gruppen mit vier bis zehn Ringkohlenstoffatomen und mindestens einem Ringheteroatom, die jeweils unabhängig voneinander substituiert sein können. Beispiele für Heteroatome sind Sauerstoff, Stickstoff, Phosphor, Bor, Selen oder Schwefel. Beispiele für Heterocyclylengruppen sind Furandiyl, Thiophendiyl, Pyrroldiyl oder Imidazoldiyl. Heterocyclylengruppen sind vorzugsweise aromatisch.

Aralkylengruppen sind typischerweise Arylgruppen, an die ein oder zwei Alkylgruppen kovalent gebunden sind. Aralkylgruppen können über ihren Arylrest und ihren Alkylrest oder über zwei Alkylreste mit dem Rest des Moleküls kovalent verbunden sein. Die Aralkylengruppe kann am aromatischen Ring beispielsweise mit Alkylgruppen oder mit Halogenatomen substituiert sein. Beispiele für Aralkylengruppen sind Benzylen oder Dimethylphenylen (Xylylen).

Beispiele für dreiwertige organische Reste R⁸ sind Alkyltriyl, Alkoxytriyl, Tris-poly(alkylenoxy), Tris-poly(alkylenamino), Cycloalkyltriyl, Aryltriyl, Aralkyltriyl oder Heterocyclyltriyl. Diese Reste entsprechen den bereits weiter oben eingehend beschriebenen zweiwertigen Resten mit dem Unterschied, dass diese mit drei kovalenten Bindungen anstelle von zwei kovalenten Bindungen mit dem Rest des Moleküls verbunden sind.

Beispiele für vierwertige organische Reste R⁸ sind Alkylquaternyl, Alkoxyquaternyl, Quater-poly(alkylenoxy), Quater-poly(alkylenamino), Cycloalkylquaternyl, Arylquaternyl, Aralkylquaternyl oder Heterocyclylquaternyl. Diese Reste entsprechen den bereits weiter oben eingehend beschriebenen zweiwertigen Resten mit dem Unterschied, dass diese mit vier kovalenten Bindungen anstelle von zwei kovalenten Bindungen mit dem Rest des Moleküls verbunden sind.

Beispiele für fünfwertige organische Reste R⁸ sind Alkylquinquinyl, Alkoxyquinquinyl, Quinqui-poly(alkylenoxy), Quinquipoly(alkylenamino), Cycloalkylquinquinyl, Arylquinquinyl, Aralkylquinquinyl oder Heterocyclylquinquinyl. Diese Reste entsprechen den bereits weiter oben eingehend beschriebenen zweiwertigen Resten mit dem Unterschied, dass diese mit fünf kovalenten Bindungen anstelle von zwei kovalenten Bindungen mit dem Rest des Moleküls verbunden sind.

Beispiele für sechswertige organische Reste R⁸ sind Alkylhexyl, Alkoxyhexyl, Hexylpoly(alkylenoxy), Hexyl-poly(alkylenamino), Cycloalkylhexyl, Arylhexyl, Aralkylhexyl oder Heterocyclylhexyl. Diese Reste entsprechen den bereits weiter oben eingehend beschriebenen zweiwertigen Resten mit dem Unterschied, dass diese mit sechs kovalenten Bindungen anstelle von zwei kovalenten Bindungen mit dem Rest des Moleküls verbunden sind.

Die Verbindungen Formel I und der weiter unten definierten Formel II können nach Durchführung eines Austauschs der Chloridanionen oder der Sulfatanionen beliebige Gegenionen X^{a-} aufweisen. Dabei kann es sich um anorganische oder um organische a-wertige Anionen oder um Gemische solcher Anionen handeln.

Beispiele für anorganische Anionen X^{a-} sind Halogenidionen, wie Fluorid, Chlorid, Bromid oder lodid, oder Hydroxidionen oder Anionen anorganischer Säuren, wie Phosphat, Sulfat, Nitrat, Borat, Hexafluorophosphat, Tetrafluoroborat, Perchlorat, Chlorat, Hexafluoroantimonat, Hexafluoroarsenat, Cyanid. Zu den Sulfat- oder Phospatanionen zählen auch solche, die einen organischen Rest aufweisen, insbesondere einen Alkylrest, ganz besonders bevorzugt einen Methylrest. Bevorzugt wird das Methylsulfatanion CH₃-OSO₃⁻.

Beispiele für organische Anionen X^{a-} sind Anionen ein- oder mehrwertiger Carbonsäuren oder ein- oder mehrwertiger Sulfonsäuren, wobei diese Säuren gesättigt oder ungesättigt sein können. Weitere Beispiele für organische Anionen X^{a-} sind Anionen von Polycarbonsäuren oder von Polysulfonsäuren. Beispiele für Anionen organischer Säuren sind Acetat, Formiat, Trifluoroacetat, Trifluormethansulfonat, Pentafluorethansulfonat, Nonofluorbutansulfonat, Butyrat, Citrat, Fumarat, Glutarat, Lactat, Malat, Malonat, Oxalat, Pyruvat oder Tartrat. Beispiele für Anionen von Polycarbonsäuren sind Polyacrylat- oder Polymethacrylatanionen. Ein Beispiel für Anionen von Polysulfonsäuren ist Polystyrensulfonat (PSS).

Bevorzugte Anionen X^{a-} sind Halogenidionen, Hydroxyionen, Phosphationen, Sulfationen, Perchlorationen, Hexafluorophosphationen oder Tetrafluoroborationen.

Die Wertigkeit der Anionen X^{a-} beträgt 1 bis 10.000, vorzugsweise 1 bis 50, und bei Verbindungen der Formel I besonders bevorzugt 1 bis 3, insbesondere 1 oder 2 und ganz besonders bevorzugt 1, sowie bei Strukturen der Formel II besonders bevorzugt 2 bis 5.

Besonders bevorzugt ist X^{a-} Chlorid oder ein Sulfatanion, ganz besonders bevorzugt Chlorid oder ein Methylsulfatanion.

Bei dem in Schritt d) oder a") verwendeten aprotischen Lösungsmittel handelt es sich um ein nichtprotisches organisches Lösungsmittel. Dabei kann es sich um aprotisch-unpolare organische Lösungsmittel, um aprotisch-polare organische Lösungsmittel oder um Gemische aus aprotisch-unpolaren organischen Lösungsmitteln und aus aprotisch-polaren organischen Lösungsmitteln handeln. Bevorzugt werden Gemische aus aprotisch-unpolaren organischen Lösungsmitteln und aus aprotisch-polaren organischen Lösungsmitteln eingesetzt.

Beispiele für aprotisch-unpolare organische Lösungsmittel sind Alkane, wie Hexan, Octan, Decan, Cyclohexan oder Petrolether; oder Alkene, wie Hexen oder Cyclohexen; oder aromatische Kohlenwasserstoffe, wie Benzol oder mit aliphatischen Resten substituiertes Benzol, z.B. Toluol oder Xylole; oder Carbonsäureester, wie Ethylacetat; oder Ether, z. B. Diethylether; oder völlig symmetrische Organosilane, wie Tetramethylsilan; oder völlig symmetrische Halogenkohlenwasserstoffe, wie Tetrachlorkohlenstoff; oder halogenierte Kohlenwasserstoffe, die trotz der hohen Elektronegativität des betreffenden Halogens nur wenig polar sind, wie Methylenchlorid oder die perfluorierten Kohlenwasserstoffe, wie Hexafluorbenzol.

Beispiele für aprotisch-polare organische Lösungsmittel sind Verbindungen mit stark polaren funktionellen Gruppen, wie der Carbonylgruppe, der Nitrogruppe oder der Nitrilgruppe, so dass diese Verbindungen ein hohes Dipolmoment aufweisen. Dazu zählen beispielsweise Ketone, z. B. Aceton; oder Lactone, wie γ-Butyrolacton; oder Lactame, wie N-Methyl-2-pyrrolidon; oder Nitrile, wie Acetonitril; oder Nitroverbindungen, wie Nitromethan; oder tertiäre Carbonsäureamide, wie Dimethylformamid; oder Harnstoffderivate, wie Tetramethylharnstoff oder Dimethylpropylenharnstoff (DMPU); oder Sulfoxide, wie Dimethylsulfoxid (DMSO); oder Sulfone, wie Sulfolan; oder Kohlensäureester, wie Dimethylcarbonat oder Ethylencarbonat.

Besonders bevorzugt wird als aprotisches Lösungsmittel in Schritt d) oder in Schritten a), a') oder a") ein organisches Nitril und/oder ein aromatischer Kohlenwasserstoff eingesetzt, insbesondere Acetonitril und/oder Toluol.

Die reduktive Aminierung in Schritt b) wird bei Temperaturen zwischen 0 und 200°C durchgeführt. Typische Temperaturen liegen im Bereich von 0 °C bis 200 °C, vorzugsweise von 20 °C bis 150 °C, und ganz besonders bevorzugt bei 40 °C bis 100 °C. Typische Drucke liegen im Bereich von 1 bis 500 bar, bevorzugt von 1 bis 300 bar, vorzugsweise von 1 bar bis 100 bar, und ganz besonders bevorzugt von 2 bar bis 50 bar.

Als Reduktionsmittel können in Schritt b) Wasserstoff, Alkalimetalle, Magnesium, Aluminium, Zink, Alkalialuminiumhydride, Alkaliborhydride, Alkalihydride, Erdalkalihydride, Alkalisulfite, Alkalidithioite, Alkalithiosulfate oder Hydrazin verwendet werden.

Besonders bevorzugt wird Wasserstoff in Schritt b) verwendet.

Beispiele für Alkalimetalle sind Lithium, Natrium oder Kalium.

Beispiele für Alkalialuminiumhydride sind Lithiumaluminiumhydrid, Natriumaluminiumhydrid oder Kaliumaluminiumhydrid.

Beispiele für Alkaliborhydride sind Lithiumborhydrid, Natriumborhydrid oder Kaliumborhydrid.

Beispiele für Alkalihydride sind Lithiumhydrid, Natriumhydrid oder Kaliumhydrid.

Beispiele für Erdalkalihydride sind Magnesiumhydrid oder Calciumhydrid.

Beispiele für Alkalisulfite sind Natriumsulfit oder Kaliumsulfit.

Beispiele für Alkalidithionite sind Natriumdithionit oder Kaliumdithionit.

Beispiele für Alkalithiosulfate sind Natriumthiosulfat oder Kaliumthiosulfat.

Die Hydrierung in Schritt b) erfolgt in Gegenwart eines Hydrierkatalysators.

Beispiele für geeignete Hydrierkatalysatoren sind Nickel, das üblicherweise als Raney-Nickel oder als Nickel geträgert auf Kieselgur eingesetzt wird, oder Kobalt, das üblicherweise als Raney-Kobalt eingesetzt wird, oder Eisen, Kupferchromit, Zinkchromit und ganz besonders bevorzugt Platinmetalle.

Als Platinmetall kommen Ruthenium, Rhodium, Palladium, Osmium, Iridium, und Platin in Frage. Besonders bevorzugt werden Palladium und Platin eingesetzt.

Vorzugsweise werden Palladium und/oder Platin als Metall oder Oxid auf verschiedenen Trägermaterialien aufgezogen eingesetzt.

Beispiele für bevorzugt eingesetzte Platin- oder Palladiumkatalysatoren sind Platinmohr, Adams-Katalysator (Platin(IV)-oxid), Platin auf Aktivkohle, Palladium auf Aktivkohle, kolloides Palladium, Palladium(II)-oxid, Palladium auf Bariumsulfat, Palladium(II)-hydroxid auf Bariumsulfat, Lindlar-Katalysator (Palladium auf Calciumcarbonat und mit Blei(II)-acetat vergiftet), Palladium auf Calciumcarbonat oder Palladiummohr.

Der Katalysator kann in Form eines Salzes oder Komplexes des betreffenden Metalls oder als feinverteiltes Metall eingesetzt werden. Es lassen sich auch Legierungen von Platinmetallen mit anderen Metallen verwenden. Vorzugsweise werden geträgerte Katalysatoren verwendet.

Als Katalysatoren lassen sich handelsübliche geträgerte Platinmetall enthaltende Katalysatoren einsetzen. Der Metallgehalt dieser Katalysatoren beträgt üblicherweise 0,3 - 10 Gew. %, bevorzugt 0,3 - 8 Gew. %, und besonders bevorzugt 0,5 - 5 Gew. %. Dabei sind die Prozentangaben auf die Trockenmasse des geträgerten Katalysators bezogen.

Als Träger kommen einen Vielzahl von Materialien in Betracht. Beispiele dafür sind Aluminiumoxid, keramische Trägermaterialien oder Kohlenstoff bzw. Graphit, beispielsweise Aktivkohle. Trägermaterialien für diese Katalysatoren sind dem Fachmann bekannt und werden in der Regel in fein verteilter Form verwendet, die ggf. zu Pellets verpresst sein können. Besonders bevorzugt wird Kohlenstoff, insbesondere Aktivkohle als Trägermaterial eingesetzt. Die Katalysatoren können als Feststoffe, z.B. als Pulver oder Pellets eingesetzt werden, aber auch als handelsübliche wasserfeuchte Paste.

Das katalytisch aktive Metall ist ein Metall der Platingruppe oder eine Kombination von Platinmetall mit anderen Metallen, beispielsweise mit Palladium oder Rhodium.

Der Katalysator kann neben dem katalytisch aktiven Metall noch mit weiteren Komponenten dotiert sein, beispielsweise mit Alkali- oder Erdalkalimetallen und/oder mit seltenen Erden.

Ganz besonders wird ein auf Kohlenstoff geträgerter Platin-Katalysator eingesetzt. Es können beliebige Kohleträger eingesetzt werden. Diese Katalysatoren sind handelsüblich.

Ein weiterer bevorzugt eingesetzter Hydrierkatalysator ist Raney-Nickel.

Die molare Menge an eingesetztem Metall, bezogen auf das eingesetzte Amin oder auf das eingesetzte Imin, liegt in der Regel zwischen 1 * 10⁻⁷ und 1 * 10⁻¹ Moläquivalenten, bevorzugt zwischen 1 * 10⁻⁶ und 5 * 10⁻² Moläquivalenten und besonders bevorzugt zwischen 5 * 10⁻⁴ und 1 * 10⁻² Moläquivalenten.

Der Katalysator liegt im Reaktionsgemisch suspendiert vor, die Reaktanten werden im Lösungsmittel gelöst und bilden eine flüssige Phase, die mit dem in der Gasphase vorliegenden Wasserstoff und/oder mit dem in der Reaktionslösung vorliegenden Reduktionsmittel reagiert.

Die Umsetzung des Reaktionsgemisches in Schritt e) erfolgt bei den oben für Schritt b) genannten Temperaturen und Drucken. Typische Temperaturen für Schritt e) liegen im Bereich von 0 °C bis 200 °C, vorzugsweise von 25 °C bis 150 °C, und ganz besonders bevorzugt bei 40 °C bis 100 °C. Typische Drucke für Schritt e) liegen im Bereich von 1 bar bis 250 bar, vorzugsweise von 1 bar bis 100 bar, und ganz besonders bevorzugt bei 1 bar bis 25 bar.

Schritt e) kann in An- und Abwesenheit einer Base durchgeführt werden. Insbesondere bei 4-Amino-alkylpiperidinylverbindungen der Formel VII, in denen die 4-Aminogruppe ein oder mehrere Wasserstoffatome aufweist, ist es bevorzugt, wenn die Umsetzung in Schritt e) in Anwesenheit einer Base durchgeführt wird.

Beispiele für geeignete Basen sind anionische Ionenaustauscher, Alkalihydroxide, wie Natriumhydroxid oder Kaliumhydroxid, oder organische Amine, vorzugsweise solche Amine, die bei den Drucken und Temperaturen in Schritt e) in flüssiger Phase vorliegen.

Eine besonders bevorzugte Verfahrensvariante umfasst die Schritte a) bis e) und g) oder a) bis g).

Besonders bevorzugt werden durch das erfindungsgemäße Verfahren Verbindungen der Formel I hergestellt.

Die erfindungsgemäß hergestellten Verbindungen der Formel I oder II können durch Oxidation in die entsprechenden 4-Ammonium-alkylpiperidin-1-yloxylsalze überführt werden.

Die Erfindung betrifft daher auch ein Verfahren zur Herstellung von 4-Ammonium-alkylpiperidin-1-yloxylsalze der Formel III oder der Formel IV 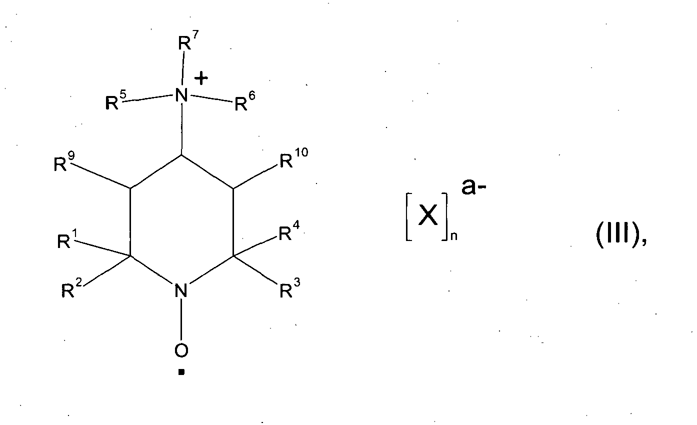 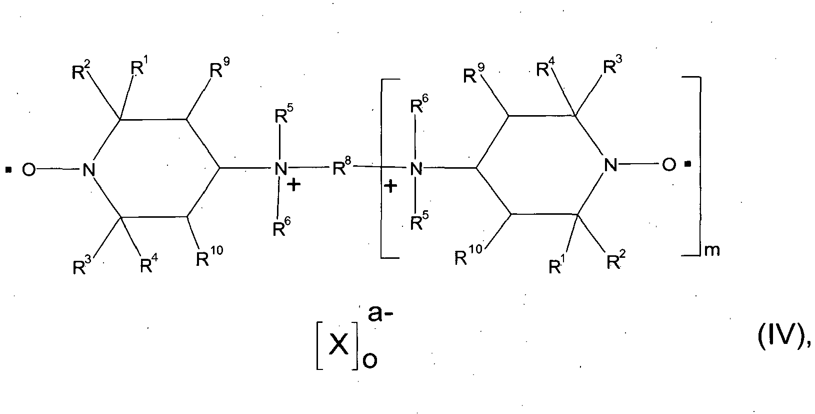
worin R¹, R², R³ und R⁴ C₁-C₆-Alkyl sind,
R⁹ und R¹⁰ unabhängig voneinander Wasserstoff oder C₁-C₆-Alkyl bedeuten,
R⁵ und R⁶ unabhängig voneinander Wasserstoff, Alkyl, Cycloalkyl, Aryl, Aralkyl oder Heterocyclyl bedeuten, die gegebenenfalls mit ein oder mehreren Resten ausgewählt aus der Gruppe bestehend aus Alkyl, Alkoxy, Hydroxy, Carboxyl, Carboxylester, Carboxylamid, Sulfonat, Sulfonsäureester, Sulfonamid oder Halogen substituiert sind und/oder die gegebenenfalls in der Alkylgruppe durch ein oder mehrere, nicht direkt zueinander benachbarte zweiwertige Reste ausgewählt aus der Gruppe bestehend aus Sauerstoff oder Imino unterbrochen sind, oder in denen R⁵ und R⁶ zusammen eine Alkylengruppe bilden,
R⁷ Alkyl bedeutet, das gegebenenfalls mit ein oder mehreren Resten ausgewählt aus der Gruppe bestehend aus Alkyl, Alkoxy, Hydroxy, Nitro, Carboxyl, Carboxylester, Carboxylamid, Sulfonat, Sulfonsäureester, Sulfonamid oder Halogen substituiert ist und/oder das gegebenenfalls in der Alkylgruppe durch ein oder mehrere, nicht direkt zueinander benachbarte zweiwertige Reste ausgewählt aus der Gruppe bestehend aus Sauerstoff oder Imino unterbrochen ist,
R⁸ eine zwei- bis sechswertige organische Brückengruppe ist, die gegebenen-falls mit ein oder mehreren Resten ausgewählt aus der Gruppe bestehend aus Alkyl, Alkoxy, Hydroxy, Nitro, Carboxyl, Carboxylester, Carboxylamid, Sulfonat, Sulfonsäureester, Sulfonamid oder Halogen substituiert ist und/oder die gegebenenfalls in der Alkylengruppe durch ein oder mehrere, nicht direkt zueinander benachbarte zweiwertige Reste ausgewählt aus der Gruppe bestehend aus Sauerstoff oder Imino unterbrochen ist,
a eine ganze Zahl von 1 bis 10.000 bedeutet,
m eine ganze Zahl von 1 bis 5 bedeutet,
n eine Zahl mit dem Wert 1/a bedeutet,
o eine Zahl mit dem Wert (1 + m) / a bedeutet, und
X ein organisches oder anorganisches Anion ist, vorzugsweise ein Chloridanion oder ein Sulfatanion ist,
wobei das Verfahren die folgende Maßnahme umfasst:
   h) Umsetzen des Reaktionsproduktes aus dem weiter oben definierten Schritt e), f) oder g) mit einem Peroxid.

Als Peroxid kann eine beliebige anorganische oder organische Perverbindung eingesetzt werden. Beispiele für organische Perverbindungen sind Peroxycarbonsäuren, organische Peroxide oder organische Hydroperoxide. Beispiele für anorganische Peroxide sind ionische Peroxidsalze und anorganische Säureperoxide, die kovalent gebundene Peroxideinheiten tragen. Dazu zählen insbesondere die Peroxide der Alkali- und Erdalkalimetalle, Wasserstoffperoxid und Peroxide von Mineralsäuren. Bevorzugt wird Wasserstoffperoxid eingesetzt.

In einer weiteren bevorzugten Variante des erfindungsgemäßen Verfahrens wird in Schritt h) das Reaktionsprodukt aus Schritt e) direkt eingesetzt.

In einer weiteren bevorzugten Variante des erfindungsgemäßen Verfahrens wird das Reaktionsprodukt aus Schritt e) mit Alkalihydroxid und Katalysator versetzt und dann mit Wasserstoffperoxid behandelt.

Ganz besonders bevorzugt wird das Reaktionsprodukt aus Schritt e) mit Alkalihydroxid und Natriumwolframat (Na₂(WO₄)) versetzt und dann mit Wasserstoffperoxid behandelt oder das Reaktionsprodukt aus Schritt e) wird mit Erdalkalisulfat versetzt und dann mit Wasserstoffperoxid behandelt..

Die erfindungsgemäß hergestellten Verbindungen der Formel III und IV können vielseitig eingesetzt werden. Beispiele dafür sind die Verwendung als Katalysator, als Kontrastmittel in bildgebenden Verfahren oder als Energiespeicher, vorzugsweise als redoxaktives Material in Batterien und Redox-Flow-Batterien.

Die nachfolgenden Beispiele erläutern die Erfindung, ohne diese zu begrenzen.

### Beispiel 1: Synthese von TEMPTMA

### Syntheseschritte:

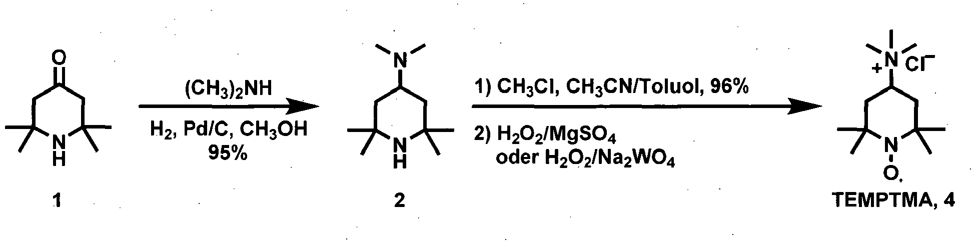

### Synthese von N,N,2,2,6,6-Hexamethylpiperidin-4-amin (2)

4-Oxo-2,2,6,6-tetramethylpiperidin (**1**, 98,4 g, 634 mmol) wurde in Methanol (300 mL) gelöst. Die Lösung wurde in einen Büchi ecoclave Druckreaktor überführt und auf aktivierter Holzkohle geträgertes Palladium (10% Pd; 3,6 g) und Dimethylamin (57 g, 1,27 mol) wurden langsam bei -10 °C zugegeben. Anschließend wurde Wasserstoff dem Reaktionsgemisch bei 60 °C zugegeben (maximaler Druck 3 bar) bis gemäß GC-MS-Analyse die vollständige Umwandlung des Ketons abgeschlossen war. Nach dem Abkühlen auf Raumtemperatur wurde der Katalysator durch Filtration entfernt. Durch destillative Abtrennung des Lösungsmittels wurde das Rohprodukt erhalten (110,9 g, 95%) und direkt in der nächsten Stufe eingesetzt.

¹H NMR-Analysedaten (300 MHz, D₂O): δ=0.75-1.15 (m, 14H; CH₃, CH₂), 1.6 (dd, ²*J*(H,H)=12.6, ³*J*(H,H)=3.2 Hz, 2H; CH₂), 2.03 (s, 6H; N-CH₃), 2.56 ppm (tt, ³*J*(H,H)=3.2, 12.2 Hz, 1H; N-CH); ¹³C NMR (75 MHz, D₂O): δ=26.7 (CH₃), 33.4 (CH₃), 39.6 (CH₂), 39.8 (N-CH₃), 50.8 (C_{q}), 55.5 ppm (C-N); ATR-FTIR (*v*): 651 (s), 690 (s), 783 (m), 856 (m), 1029 (s), 1103 (m), 1242 (s), 1323 (w), 1365 (s), 1454 (s), 2927 (w), 2951 cm⁻¹ (w); HRMS (ESI, *m*/*z*): [*M*]⁺ berechnet für [C₁₁H₂₄N₂]^{H+}, 185.2012; gefunden, 185.2011.

### Synthese von N,N,N,2,2,6,6-Heptamethylpiperidinyloxy-4-ammoniumchlorid (TEMPTMA, 4)

A) Das in der vorangegangenen Stufe erhaltene Amin **2** (109 g, 591 mmol) wurde in Acetonitril/Toluol (1:1; 200 mL) aufgelöst und die Lösung wurde in einen Büchi ecoclave Druckreaktor übergeführt. Bei 70°C wurde portionsweise Chlormethan hinzugefügt, wobei der Druck unter 3 bar gehalten wurde, bis kein weiterer Verbrauch des Gases beobachtet wurde (ca. 60 g, 1,2 mol). Nach Abkühlen auf 20 °C wurde der Niederschlag auf einer Glasfritte gesammelt und mit Toluol gewaschen (2 × 100 mL). Nachfolgend wurde getrocknet (40 °C, 10 mbar) und es wurde das Produkt **3** als weißer Feststoff erhalten (133,7 g, 96%).
B) Oxidation von **3**
   Zu einer Lösung von **3** (90 g, 383 mmol), Natriumhydroxid (3 g, 75 mmol, pH∼11) und Magnesiumsulfat (2,3 g, 19.2 mmol) in Wasser (120 mL) wurden bei 90 °C tropfenweise Wasserstoffperoxid (30 Gew.-%; 117 mL, 1,15 mol) hinzugefügt, bis eine dünnschichtchromatographische Analyse (Acetonitril/Wasser/KNO₃: 10/1/0,1) eine quantitative Umsetzung anzeigte. Anschließend wurde der Niederschlag durch Filtration entfernt, der *pH-Wert* der Lösung auf 5 eingestellt, und die Lösung bei 90 °C gerührt, um Spuren von Wasserstoffperoxid zu entfernen. Das gewünschte Produkt **4** wurde nach destillativer Entfernung des Wassers und Trocknen bei 40 °C (10 mbar) als orangener Feststoff erhalten (96 g, quantitativ). Spuren von Salz wurden nicht entfernt, da diese bei der elektrochemischen Anwendung nicht stören.
   ¹H NMR-Analyse (300 MHz, D₂O): δ=1.09 (m, 12H; CH₃), 1.60 (pt, *J*(H,H)=12.2, 2H; CH₂), 2.06 (pd, *J*(H,H)=11.3, 2H; CH₂), 2.95 (s, 9H; N-CH₃), 3.59 ppm (t, *J*(H,H)= 12.6 Hz, 1H; N-CH), (abgelöscht mit Phenylhydrazin); ATR-FTIR (v): 671 (m), 786 (w), 848 (w), 902 (s), 948 (m), 964 (m), 983 (m), 1110 (m), 1184 (s),1246 (m), 1269 (w), 1346 (m), 1477 (m), 1496 (m), 2970 (w), 3020 cm⁻¹ (w).HRMS (ESI, *m*/*z*): [*M*]⁺ berechnet für [C₁₂H₂₆ClN₂O]^{-Cl}, 214.2040; gefunden, 214.2042; ESR: *g*=2.0061.

### Beispiel 2: Synthese von N-(methoxymethyl)-N,N,2,2,6,6-hexamethylpiperidin-4-ammoniumchlorid bei Normaldruck

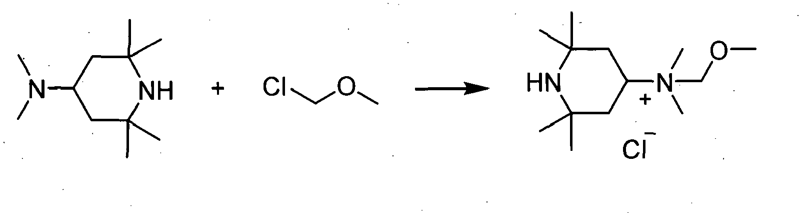

*N*,*N*,2,2,6,6-Hexamethylpiperidin-4-amin (**2**, 100 mg, 0,5 mmol) wurde in Acetonitril/Toluol (1:1; 1 mL) aufgelöst und Chlor(methoxy)methan (44 mg, 0,5 mmol) hinzugefügt. Nach 5 Stunden Rühren bei 40 °C und Normaldruck wurde auf 20 °C abgekühlt, der geformte Niederschlag auf einer Glasfritte gesammelt und mit Toluol gewaschen. Nachfolgend wurde getrocknet (40 °C, 10 mbar) und das Produkt als weißer Feststoff erhalten (30 mg).

¹H NMR-Analyse (300 MHz, D₂O): δ=1.10 (m, 12H; CH₃), 1.33 (pt, *J*(H,H)=12.2, 2H; CH₂), 1.95 (pd, *J*(H,H)=14.5, 2H; CH₂), 2.87 (s, 6H; N-CH₃), 3.56 (s, 3H; O-CH₃), 3.80 ppm (pt, *J*(H,H)= 12.6 Hz, 1H; N-CH), 4.56 (s, 2H; -CH₂-O); ¹³C NMR (75 MHz, D₂O): δ=26.3 (CH₃), 32.9 (CH₃), 36.8 (CH₂), 44.7 (N-CH₃), 51.7 (C_{q}), 60.4 (C-N), 64.9 (O-CH₃), 90.5 ppm (O-CH₂).

### Beispiel 3: Synthese von N,N,2,2,6,6-Hexamethyl-N-propylpiperidin-4-ammoniumchlorid

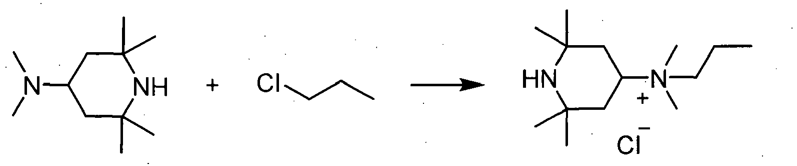

*N*,*N*,2,2,6,6-hexamethylpiperidin-4-amin (**2**, 100 mg, 0,5 mmol) wurde in 1-Chlorpropan (1 mL) aufgelöst und sechs Stunden in einem Druckgefäß im Mikrowellenofen bei 140 °C und 6 bar gerührt. Nach dem Abkühlen auf 20 °Cwurde der geformte Niederschlag abzentrifugiert und mit Toluol gewaschen. Nachfolgend wurde getrocknet (40 °C, 10 mbar) und das Produkt als weißer Feststoff erhalten (25 mg).

¹H NMR-Analyse (300 MHz, D₂O): δ=0.85 (t, J(H,H)=7.2, 3H; CH₂₋C*H₃*), 1.18 (m, 12H; CH₃), 1.46 (pt, *J*(H,H)=12.1, 2H; CH₂), 1.68 (m, 2H; C*H₂*-CH₃), 2.05 (pd, *J*(H,H)=10.3, 2H; CH₂), 2.92 (s, 6H; N-CH₃), 3.19 (m, 2H; N-CH₂), 3.68 ppm (pt, *J*(H,H)= 12.6 Hz, 1H; N-CH₃); ¹³C NMR (75 MHz, D₂O): δ=9.9 (CH₃), 15.5 (CH₂), 25.6 (CH₃), 32.2 (CH₃), 36.3 (CH₂), 47.8 (N-CH₃), 53.1 (C_{q}), 64.4 (CH₂-N), 66.9 ppm (CH-N).

### Beispiel 4: Synthese von N,N,N,2,2,6,6-Heptamethylpiperidinyl-4-ammoniumchlorid bei Normaldruck

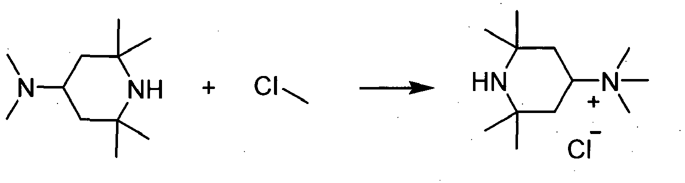

*N*,*N*,2,2,6,6-Hexamethylpiperidin-4-aminiumchlorid (**2**, 100 mg, 0,5 mmol) wurde in Acetonitril/Toluol (1:1; 1 mL) aufgelöst und in einem offenen Kolben bei Normaldruck auf 60 °C erwärmt. Unter Rühren wurde für 1,5 Stunden Chlormethan durch die Lösung geleitet. Nach dem Abkühlen auf 20 °C wurde der geformte Niederschlag abzentrifugiert und mit Toluol gewaschen. Nachfolgend wurde getrocknet (40 °C, 10 mbar) und das Produkt als weißer Feststoff erhalten (80 mg).

¹H NMR-Analyse (300 MHz, D₂O): δ=1.17 (m, 12H; CH₃), 1.36 (pt, *J*(H,H)=12.2, 2H; CH₂), 2.10 (pd, *J*(H,H)=12.1, 2H; CH₂), 3.02 (s, 9H; N-CH₃), 3.73 ppm (pt, *J*(H,H)= 12.6 Hz, 1H; N-CH₃); ¹³C NMR (75 MHz, D₂O): δ=26.2 (CH₃), 32.9 (CH₃), 37.1 (CH₂), 50.5 (N-CH₃), 51.9 (C_{q}), 69.2 ppm (CH-N).

### Beispiel 5: Synthese von N,N,N,2,2,6,6-Heptamethylpiperidinyl-4-ammoniummethylsulfat

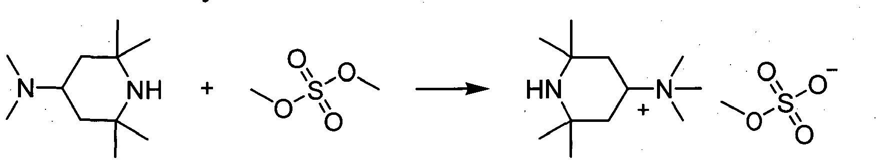

*N*,*N*,2,2,6,6-Hexamethylpiperidin-4-amin (**2**, 100 mg, 0,5 mmol) wurde in Acetonitril/- Toluol (1:1; 1 mL) aufgelöst, Dimethylsulfat (50 µL, 0,5 mmol) hinzugegeben und die Lösung zwei Stunden in einem Druckgefäß im Mikrowellenofen bei 160 °C und 8 bar gerührt. Nach dem Abkühlen auf 20 °C wurde der Ansatz bis zur Trockne eingeengt, nachfolgend im Vakuum getrocknet (40 °C, 10 mbar) und das Produkt als brauner Feststoff erhalten (120 mg).

¹H NMR-Analyse (300 MHz, D₂O): *δ*=1.23 (m, 12H; CH₃), 1.46 (pt, *J*(H,H)=12.2, 2H; CH₂), 2.17 (pd, *J*(H,H)=12.4, 2H; CH₂), 3.04 (s, 9H; N-CH₃), 3.69 (s, 3H; CH₃SO₄), 3.77 ppm (pt, *J*(H,H)= 12.6 Hz, 1H; N-CH₃); ¹³C NMR (75 MHz, D₂O): *δ*=23.5 (CH₃), 30.0 (CH₃), 36.4 (CH₂), 48.4 (N-CH₃), 50.6 (CH₃-SO₄), 53.3 (C_{q}), 66.3 ppm (CH-N).

### Beispiel 6: Synthese von N,N'-(1,4-Phenylen-bis(methylen))bis(N,N,2,2,6,6-hexamethylpiperidin-4-ammonium)chlorid

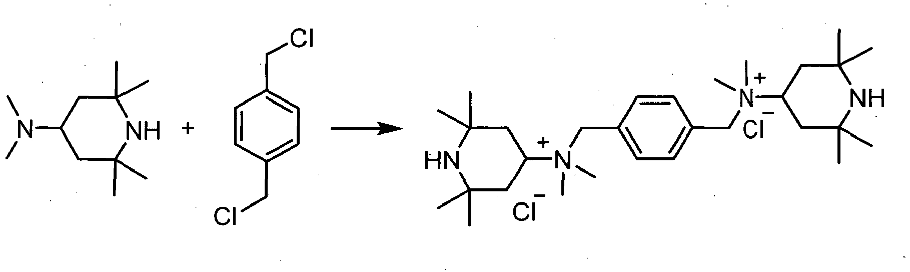

*N*,*N*,2,2,6,6-Hexamethylpiperidin-4-amin **(2,** 100 mg, 0,5 mmol) wurde in Dimethylsulfoxid (1 mL) aufgelöst und 1,4-Bis(chloromethyl)benzol (47 mg, 0,25 mmol) hinzugefügt. Nach Rühren der Lösung über sechs Stunden in einem Druckgefäß im Mikrowellenofen bei 100 °C und 1 bar wurde auf 20 °C abgekühlt, der geformte Niederschlag abzentrifugiert und mit Toluol gewaschen. Nachfolgend wurde getrocknet (40 °C, 10 mbar) und das Produkt als weißer Feststoff erhalten (110 mg).

¹H NMR-Analyse (300 MHz, D₂O): *δ*=1.11 (m, 24H; CH₃), 1.47 (pt, *J*(H,H)=12.2, 4H; CH₂), 2.18 (pd, *J*(H,H)=11.1, 4H; CH₂), 2.91 (s, 12H; N-CH₃), 3.63 ppm (pt, *J*(H,H)= 12.6 Hz, 2H; N-CH), 4.51 (s, 4H; CH₂-Ar), 7.67 ppm (s, 4H, Ar); ¹³C NMR (75 MHz, D₂O): *δ*=26.4 (CH₃), 32.8 (CH₃), 37.0 (CH₂), 47.4 (N-CH₃), 52.1 (C_{q}), 64.6 (CH₂-Ar), 67.8 (N-CH), 129.8 (C_{q,Ar}), 133.7 ppm (CH_{Ar}).

## Patentansprüche

1. Verfahren zur Herstellung von 2,2,6,6-Tetraalkylpiperidin-4-ammoniumsalzen der Formel I oder der Formel II 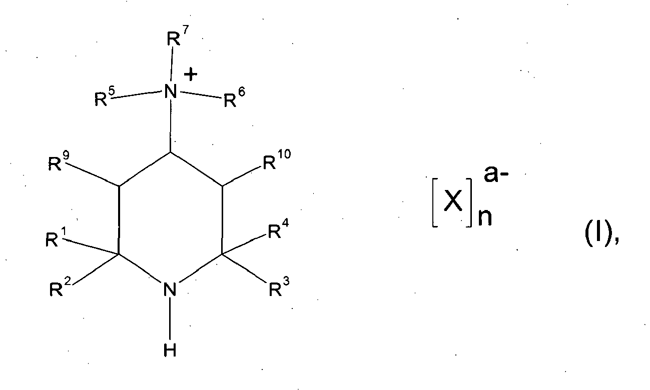 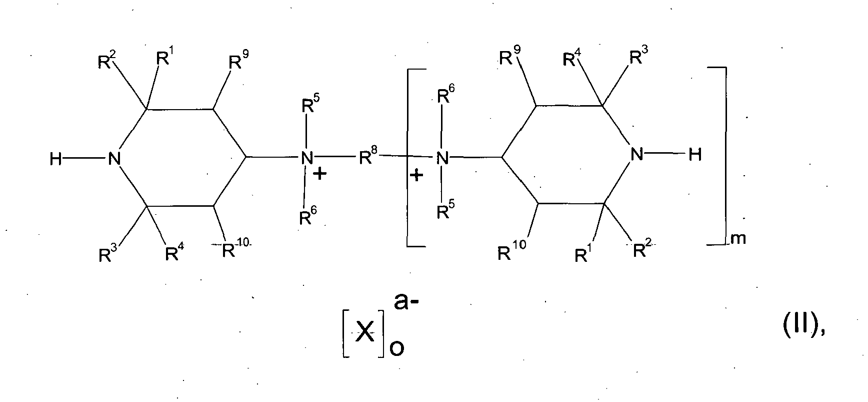
worin R¹, R², R³ und R⁴ C₁-C₆-Alkyl sind,
R⁹ und R¹⁰ unabhängig voneinander Wasserstoff oder C₁-C₆-Alkyl bedeuten,
R⁵ und R⁶ unabhängig voneinander Wasserstoff, Alkyl, Cycloalkyl, Aryl, Aralkyl oder Heterocyclyl bedeuten, oder Alkyl, Cycloalkyl, Aryl, Aralkyl oder Heterocyclyl, die mit ein oder mehreren Resten ausgewählt aus der Gruppe bestehend aus Alkyl, Alkoxy, Hydroxy, Carboxyl, Carboxylester, Carboxylamid, Sulfonat, Sulfonsäureester, Sulfonamid oder Halogen substituiert sind, oder Alkyl, das durch ein oder mehrere, nicht direkt zueinander benachbarte zweiwertige Reste ausgewählt aus der Gruppe bestehend aus Sauerstoff oder Imino unterbrochen ist, oder in denen R⁵ und R⁶ zusammen eine Alkylengruppe bilden,
R⁷ Alkyl bedeutet oder Alkyl, das mit ein oder mehreren Resten ausgewählt aus der Gruppe bestehend aus Alkyl, Alkoxy, Hydroxy, Nitro, Carboxyl, Carboxylester, Carboxylamid, Sulfonat, Sulfonsäureester, Sulfonamid oder Halogen substituiert ist, oder Alkyl, das durch ein oder mehrere, nicht direkt zueinander benachbarte zweiwertige Reste ausgewählt aus der Gruppe bestehend aus Sauerstoff oder Imino unterbrochen ist,
R⁸ eine zwei- bis sechswertige organische Brückengruppe ist, oder eine zweibis sechswertige organische Brückengruppe, die mit ein oder mehreren Resten ausgewählt aus der Gruppe bestehend aus Alkyl, Alkoxy, Hydroxy, Nitro, Carboxyl, Carboxylester, Carboxylamid, Sulfonat, Sulfonsäureester, Sulfonamid oder Halogen substituiert ist, oder eine Alkylengruppe, die durch ein oder mehrere, nicht direkt zueinander benachbarte zweiwertige Reste ausgewählt aus der Gruppe bestehend aus Sauerstoff oder Imino unterbrochen ist,
a eine ganze Zahl von 1 bis 10.000 bedeutet,
m eine ganze Zahl von 1 bis 5 bedeutet,
n eine Zahl mit dem Wert 1/a bedeutet,
o eine Zahl mit dem Wert (1 + m) / a bedeutet, und
X ein organisches Anion, ein anorganisches Anion oder eine Mischung dieser Anionen ist, wobei das Verfahren die folgenden Maßnahmen umfasst:
a) Vorlage einer Lösung von 4-Oxo-alkylpiperidin der Formel VI 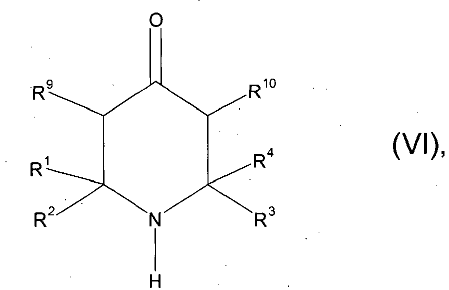 wobei R¹, R², R³, R⁴, R⁹ und R¹⁰ die oben definierte Bedeutung besitzen, und einem Amin der Formel HNR⁵R⁶, wobei R⁵ und R⁶ die oben definierte Bedeutung besitzen, in einem Lösungsmittel ausgewählt aus der Gruppe der Alkohole, Ether, Nitrile, halogenierten Kohlenwasserstoffe, aromatischen Kohlenwasserstoffe, aliphatischen Kohlenwasserstoffe oder Gemischen davon,
b) reduktive Aminierung der 4-Oxo-alkylpiperidinylverbindung der Formel VI mittels Wasserstoff, Alkalimetallen, Magnesium, Aluminium, Zink, Alkalialuminiumhydriden, Alkaliborhydriden, Alkalihydriden, Erdalkalihydriden, Alkalisulfiten, Alkalidithioniten, Alkalithiosulfaten oder Hydrazin in Gegenwart eines Hydrierkatalysators zu einer 4-Amino-alkylpiperidinylverbindung der Formel VII 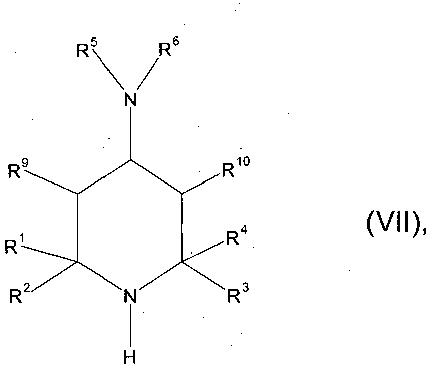 wobei R¹, R², R³, R⁴, R⁵, R⁶, R⁹ und R¹⁰ die oben definierte Bedeutung besitzen,
c) Abtrennung des Reaktionsproduktes der Formel VII aus Schritt b) vom Hydrierkatalysator und Lösungsmittel,
d) Auflösen des in Schritt c) abgetrennten Reaktionsproduktes der Formel VII in einem oder mehreren aprotischen organischen Lösungsmittel und Zuführen von einem organischen Chlorid der Formel R⁷-Cl oder der Formel Cl-R⁸-(Cl)ₘ oder von einem organischen Sulfat der Formel R⁷-O-SO₂-O-R⁷, worin R⁷, m und R⁸ die obigen Definitionen aufweisen, und
e) Umsetzen des Reaktionsgemisches aus Schritt d) bei einem Druck von 1 bis 500 bar und einer Temperatur von 0 bis 200°C,
oder, wobei das Verfahren die folgenden Maßnahmen umfasst:
a') Vorlage einer Lösung eines Imins der Formel V 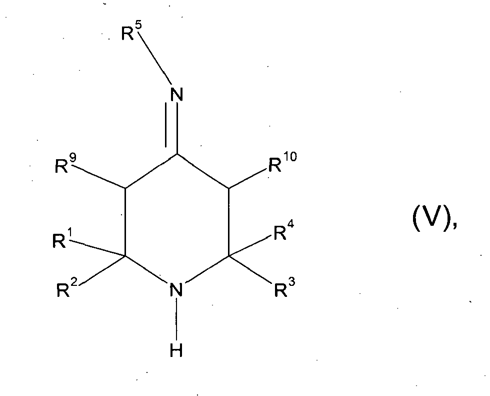 wobei R¹, R², R³, R⁴, R⁵, R⁹ und R¹⁰ die oben definierte Bedeutung besitzen, in einem Lösungsmittel ausgewählt aus der Gruppe der Alkohole, Ether, Nitrile, halogenierten Kohlenwasserstoffe, aromatischen Kohlenwasserstoffe, aliphatischen Kohlenwasserstoffe oder Gemischen davon,
b) Hydrierung des Imins der Formel V mittels Wasserstoff, Alkalimetallen, Magnesium, Aluminium, Zink, Alkalialuminiumhydriden, Alkaliborhydriden, Alkalihydriden, Erdalkalihydriden, Alkalisulfiten, Alkalidithioniten, Alkalithiosulfaten oder Hydrazin in Gegenwart eines Hydrierkatalysators zu einer 4-Amino-alkylpiperidinylverbindung der Formel VII 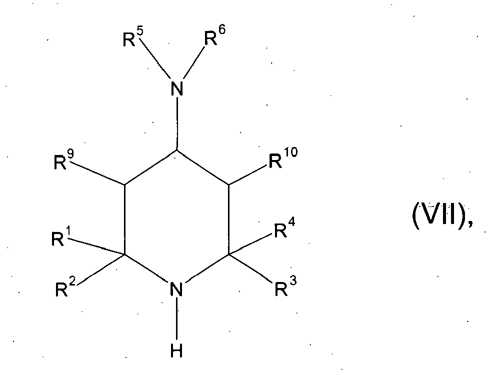 wobei R¹, R², R³, R⁴, R⁵, R⁶, R⁹ und R¹⁰ die oben definierte Bedeutung besitzen,
c) Abtrennung des Reaktionsproduktes der Formel VII aus Schritt b) vom Hydrierkatalysator und Lösungsmittel,
d) Auflösen des in Schritt c) abgetrennten Reaktionsproduktes der Formel VII in einem oder mehreren aprotischen organischen Lösungsmittel und Zuführen von einem organischen Chlorid der Formel R⁷-Cl oder der Formel Cl-R⁸-(Cl)ₘ oder von einem organischen Sulfat der Formel R⁷-O-SO₂-O-R⁷, worin R⁷, m und R⁸ die obigen Definitionen aufweisen, und
e) Umsetzen des Reaktionsgemisches aus Schritt d) bei einem Druck von 1 bis 500 bar und einer Temperatur von 0 bis 200°C,
oder, wobei das Verfahren die folgenden Maßnahmen umfasst:
a") Vorlage von 4-Amino-alkylpiperidin der Formel VII 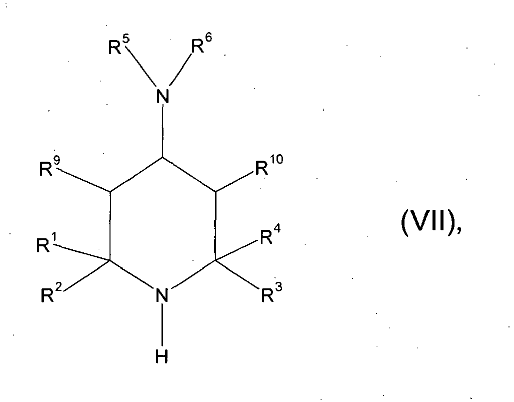 wobei R¹, R², R³, R⁴, R⁵, R⁶, R⁹ und R¹⁰ die oben definierte Bedeutung besitzen, in einem oder mehreren aprotischen organischen Lösungsmittel,
d') Zuführen von einem organischen Chlorid der Formel R⁷-Cl oder der Formel Cl-R⁸-(Cl)ₘ oder von einem organischen Sulfat der Formel R⁷-O-SO₂-O-R⁷, worin R⁷, m und R⁸ die obigen Definitionen aufweisen, zur Lösung aus Schritt a"), und
e) Umsetzen des Reaktionsgemisches aus Schritt d') bei einem Druck von 1 bis 500 bar und einer Temperatur von 0 bis 200°C.

2. Verfahren nach Anspruch 1, **dadurch gekennzeichnet, dass** nach Schritt e) als Schritt f) ein Austausch des Chloridions oder des Sulfations durch ein anderes Anion erfolgt, oder dass nach Schritt e) als Schritt g) ein Abtrennen des Reaktionsproduktes aus dem Reaktionsgemisch erfolgt, oder dass nach Schritt e) als Schritt f) ein Austausch des Chloridions oder des Sulfations durch ein anderes Anion erfolgt und dass danach als Schritt g) ein Abtrennen des Reaktionsproduktes aus dem Reaktionsgemisch erfolgt, oder dass nach Schritt e) als Schritt g) ein Abtrennen des Reaktionsproduktes aus dem Reaktionsgemisch erfolgt und dass danach als Schritt f) ein Austausch des Chloridions oder des Sulfations durch ein anderes Anion erfolgt.

3. Verfahren nach mindestens einem der Ansprüche 1 bis 2, **dadurch gekennzeichnet, dass** das Umsetzen des Reaktionsgemisches in Schritt e) in Anwesenheit einer Base erfolgt.

4. Verfahren nach mindestens einem der Ansprüche 1 bis 3, **dadurch gekennzeichnet, dass** R¹, R², R³ und R⁴ Methyl oder Ethyl sind.

5. Verfahren nach mindestens einem der Ansprüche 1 bis 4, **dadurch gekennzeichnet, dass** R⁹ und R¹⁰ Wasserstoff sind.

6. Verfahren nach mindestens einem der Ansprüche 1 bis 5, **dadurch gekennzeichnet, dass** das in Schritt a) eingesetzte Amin der Formel HNR⁵R⁶ ein sekundäres Amin ist.

7. Verfahren nach mindestens einem der Ansprüche 1 bis 6, **dadurch gekennzeichnet, dass** R⁵, R⁶ und R⁷ unabhängig voneinander Alkyl bedeuten, oder Alkyl, das gegebenenfalls mit ein oder mehreren Resten ausgewählt aus der Gruppe bestehend aus Alkyl, Alkoxy, Hydroxy, Carboxyl, Carboxylester, Carboxylamid, Sulfonat, Sulfonsäureester, Sulfamid oder Halogen substituiert ist, oder Alkyl, das durch ein oder mehrere, nicht direkt zueinander benachbarte zweiwertige Reste ausgewählt aus der Gruppe bestehend aus Sauerstoff oder Imino unterbrochen ist.

8. Verfahren nach Anspruch 7, **dadurch gekennzeichnet, dass** R⁵, R⁶ und R⁷ unabhängig voneinander C₁-C₆-Alkyl bedeuten.

9. Verfahren nach mindestens einem der Ansprüche 1 bis 8, **dadurch gekennzeichnet, dass** in Schritt d) oder d') ein organisches Chlorid eingesetzt wird.

10. Verfahren nach mindestens einem der Ansprüche 1 bis 9, **dadurch gekennzeichnet, dass** der Alkohol in den Schritten a) bis c) oder a') bis c) ein aliphatischer Alkohol mit ein bis sechs Kohlenstoffatomen ist, insbesondere Methanol oder Ethanol.

11. Verfahren nach mindestens einem der Ansprüche 1 bis 10, **dadurch gekennzeichnet, dass** das aprotische Lösungsmittel in Schritt d) oder in den Schritten a), a') oder a") ein organisches Nitril und/oder ein aromatischer Kohlenwasserstoff ist, insbesondere Acetonitril und/oder Toluol.

12. Verfahren nach mindestens einem der Ansprüche 1 bis 11, **dadurch gekennzeichnet, dass** das Verfahren die Schritte a) bis e) und g) oder a) bis g) umfasst.

13. Verfahren nach mindestens einem der Ansprüche 1 bis 12, **dadurch gekennzeichnet, dass** das Verfahren die Herstellung der Verbindung der Formel I beinhaltet.

14. Verfahren zur Herstellung von 4-Ammonium-alkylpiperidin-1-yloxylsalzen der Formel III oder der Formel IV 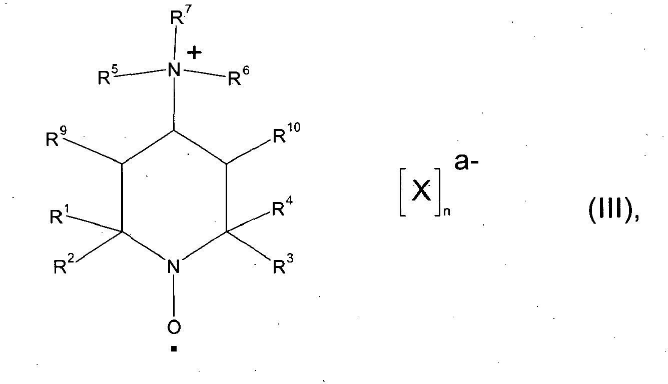 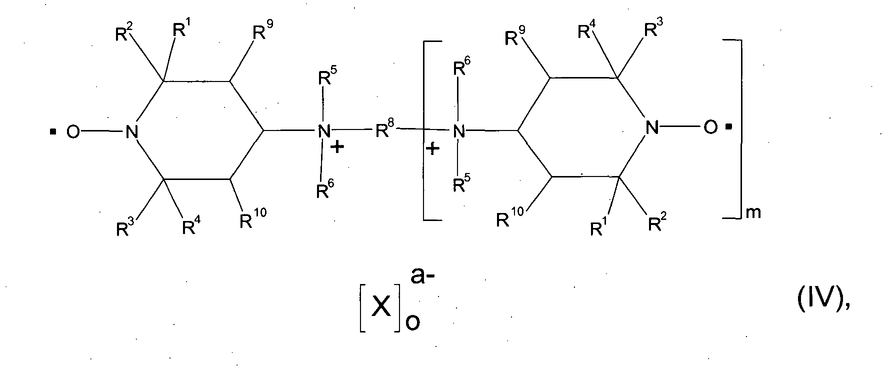
worin R¹, R², R³ und R⁴ C₁-C₆-Alkyl sind,
R⁹ und R¹⁰ unabhängig voneinander Wasserstoff oder C₁-C₆-Alkyl bedeuten,
R⁵ und R⁶ unabhängig voneinander Wasserstoff, Alkyl, Cycloalkyl, Aryl, Aralkyl oder Heterocyclyl bedeuten, oder Alkyl, Cycloalkyl, Aryl, Aralkyl oder Heterocyclyl, die mit ein oder mehreren Resten ausgewählt aus der Gruppe bestehend aus Alkyl, Alkoxy, Hydroxy, Carboxyl, Carboxylester, Carboxylamid, Sulfonat, Sulfonsäureester, Sulfonamid oder Halogen substituiert sind, oder Alkyl, das durch ein oder mehrere, nicht direkt zueinander benachbarte zweiwertige Reste ausgewählt aus der Gruppe bestehend aus Sauerstoff oder Imino unterbrochen ist, oder in denen R⁵ und R⁶ zusammen eine Alkylengruppe bilden,
R⁷ Alkyl bedeutet, oder Alkyl, das mit ein oder mehreren Resten ausgewählt aus der Gruppe bestehend aus Alkyl, Alkoxy, Hydroxy, Nitro, Carboxyl, Carboxylester, Carboxylamid, Sulfonat, Sulfonsäureester, Sulfonamid oder Halogen substituiert ist, oder Alkyl, das durch ein oder mehrere, nicht direkt zueinander benachbarte zweiwertige Reste ausgewählt aus der Gruppe bestehend aus Sauerstoff oder Imino unterbrochen ist,
R⁸ eine zwei- bis sechswertige organische Brückengruppe ist, oder eine zweibis sechswertige organische Brückengruppe, die mit ein oder mehreren Resten ausgewählt aus der Gruppe bestehend aus Alkyl, Alkoxy, Hydroxy, Nitro, Carboxyl, Carboxylester, Carboxylamid, Sulfonat, Sulfonsäureester, Sulfonamid oder Halogen substituiert ist, oder Alkyl, das durch ein oder mehrere, nicht direkt zueinander benachbarte zweiwertige Reste ausgewählt aus der Gruppe bestehend aus Sauerstoff oder Imino unterbrochen ist,
a eine ganze Zahl von 1 bis 10.000 bedeutet,
m eine ganze Zahl von 1 bis 5 bedeutet,
n eine Zahl mit dem Wert 1/a bedeutet,
o eine Zahl mit dem Wert (1 + m) / a bedeutet, und
X ein organisches oder anorganisches Anion oder eine Mischung solcher Anionen ist,
wobei das Verfahren die folgenden Maßnahmen umfasst:
Durchführung des Verfahrens mit den Schritten a) bis e) und gegebenenfalls f) und/oder g) oder mit den Schritten a') und b) bis e) und gegebenenfalls f) und/oder g) oder mit den Schritten a"), d') und e) und gegebenenfalls f) und/oder g) nach einem der Ansprüche 1 bis 13, und
h) Umsetzen des Reaktionsproduktes aus Schritt e), f) oder g) nach einem der Ansprüche 1 bis 13 mit einem Peroxid.

15. Verfahren nach Anspruch 14, **dadurch gekennzeichnet, dass** das Peroxid Wasserstoffperoxid ist.

16. Verfahren nach mindestens einem der Ansprüche 14 oder 15, **dadurch gekennzeichnet, dass** in Schritt h) das Reaktionsprodukt aus Schritt e) direkt eingesetzt wird.

17. Verfahren nach mindestens einem der Ansprüche 14 bis 16, **dadurch gekennzeichnet, dass** das Reaktionsprodukt aus Schritt e)mit Alkalihydroxid und Natriumwolframat versetzt wird und dann mit Wasserstoffperoxid behandelt wird oder dass das Reaktionsprodukt aus Schritt e) mit Erdalkalisulfat versetzt wird und dann mit Wasserstoffperoxid behandelt wird.

## Claims

1. A method for preparing of 2,2,6,6-tetraalkylpiperidine-4-ammonium salts of formula I or of formula II 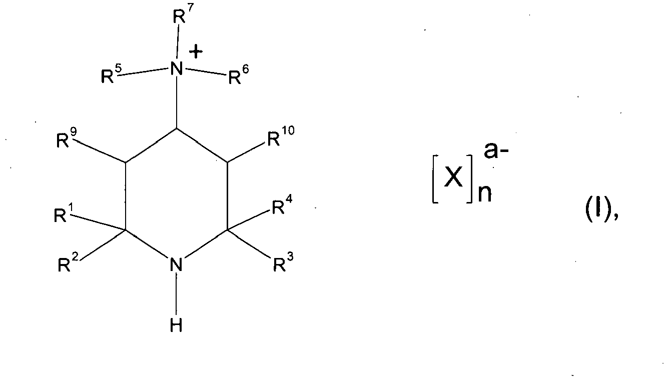 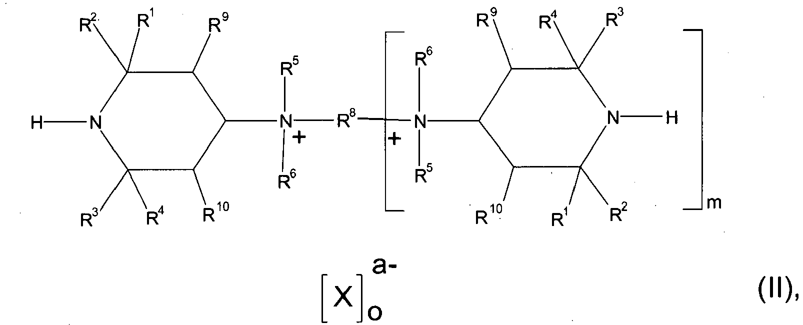
wherein R¹, R², R³ and R⁴ are C₁-C₆-alkyl,
R⁹ and R¹⁰ independently of one another are hydrogen or C₁-C₆-alkyl,
R⁵ and R⁶ independently of one another are hydrogen, alkyl, cycloalkyl, aryl, aralkyl or heterocyclyl, or alkyl, cycloalkyl, aryl, aralkyl or heterocyclyl which are substituted with one or more residues selected from the group consisting of alkyl, alkoxy, hydroxy, carboxyl, carboxylic ester, carboxylic amide, sulfonate, sulfonic acid ester, sulfonamide or halogen, or alkyl interrupted by one or more divalent residues selected from the group consisting of oxygen or imino, which are not directly adjacent to each other, or in which R⁵ and R⁶ together form an alkylene group,
R⁷ is alkyl or alkyl which is substituted with one or more residues selected from the group consisting of alkyl, alkoxy, hydroxy, nitro, carboxyl, carboxylic ester, carboxylic amide, sulfonate, sulfonic acid ester, sulfonamide or halogen, or alkyl interrupted by one or more divalent residues selected from the group consisting of oxygen or imino, which are not directly adjacent to each other,
R⁸ is a di- to sixvalent organic bridge group, or a di- to sixvalent organic bridge group, which is substituted with one or more residues selected from the group consisting of alkyl, alkoxy, hydroxy, nitro, carboxyl, carboxylic ester, carboxylic amide, sulfonate, sulfonic acid ester, sulfonamide or halogen, or an alkylene group interrupted by one or more divalent residues selected from the group consisting of oxygen or imino, which are not directly adjacent to each other,
a is an integer from 1 to 10.000,
m is an integer from 1 to 5,
n is a number with value 1/a,
o is a number with value (1 + m) / a, and
X is an organic anion, an inorganic anion or a mixture of these anions, wherein the process includes the following measures:
a) provision of a solution of 4-oxo-alkylpiperidine of formula VI 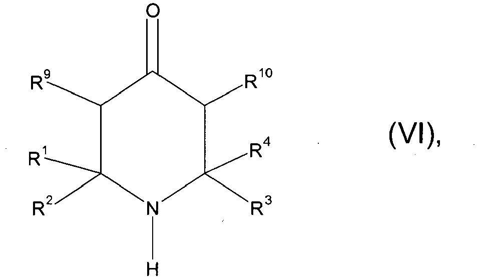 wherein R¹, R², R³, R⁴, R⁹ and R¹⁰ have the above-defined meanings, and an amine of formula HNR⁵R⁶, wherein R⁵ and R⁶ have the above-defined meanings, in a solvent selected from the group consisting of alcohols, ethers, nitriles, halogenated hydrocarbons, aromatic hydrocarbons, aliphatic hydrocarbons or mixtures thereof,
b) reductive amination of the 4-oxo-alkylpiperidinyl compound of formula VI by means of hydrogen, alkali metals, magnesium, aluminium, zinc, alkali-aluminium-hydrides, alkaliboron-hydrides, alkali hydrides, earth alkaline hydrides, alkali sulfites, alkali dithionites, alkali thiosulfates or hydrazin in the presence of a hydrogenation catalyst to a 4-amino-alkylpiperidinyl compound of formula VII. 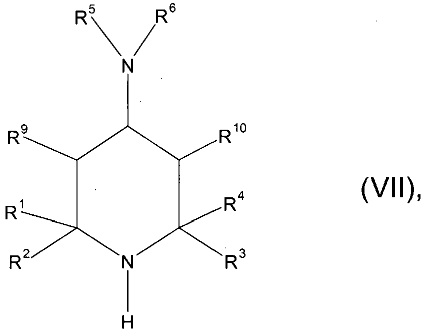 wherein R¹, R², R³, R⁴, R⁵, R⁶, R⁹ and R¹⁰ have the above-defined meaning,
c) separation of the reaction product of formula VII from step b) from the hydrogenation catalyst and the solvent,
d) dissolving the reaction product of formula VII separated in step c) in one or more aprotic organic solvents and feeding an organic chloride of formula R⁷-Cl or of formula Cl-R⁸-(Cl)ₘ or an organic sulfate of formula R⁷-O-SO₂-O-R⁷, wherein R⁷, m and R⁸ have the above meanings, and
e) reacting the reaction mixture from step d) at a pressure from 1 to 500 bar and at a temperature of 0 to 200°C,
or, wherein the process includes the following measures:
a') provision of a solution of an imine of formula V 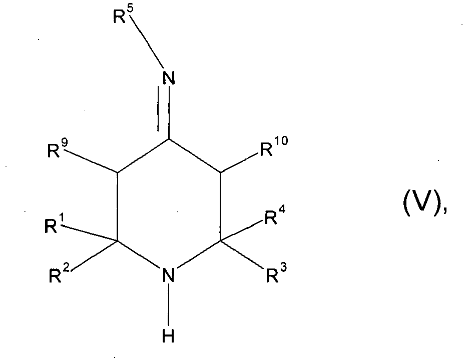 wherein R¹, R², R³, R⁴, R⁵, R⁹ and R¹⁰ have the above-defined meanings, in a solvent selected from the group consisting of alcohols, ethers, nitriles, halogenated hydrocarbons, aromatic hydrocarbons, aliphatic hydrocarbons or mixtures thereof,
b) hydrogenation of the imine of formula V by means of hydrogen, alkali metals, magnesium, aluminium, zinc, alkali-aluminium-hydrides, alkaliboron-hydrides, alkali hydrides, earth alkaline hydrides, alkali sulfites, alkali dithionites, alkali thiosulfates or hydrazin in the presence of a hydrogenation catalyst to a 4-amino-alkylpiperidinyl compound of formula VII 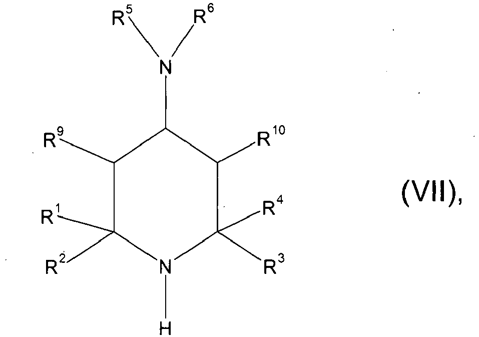 wherein R¹, R², R³, R⁴, R⁵, R⁶, R⁹ and R¹⁰ have the above-defined meaning,
c) separation of the reaction product of formula VII from step b) from the hydrogenation catalyst and the solvent,
d) dissolving the reaction product of formula VII separated in step c) in one or more aprotic organic solvents and feeding an organic chloride of formula R⁷-Cl or of formula Cl-R⁸-(Cl)ₘ or an organic sulfate of formula R⁷-O-SO₂-O-R⁷, wherein R⁷, m and R⁸ have the above meanings, and
e) reacting the reaction mixture from step d) at a pressure from 1 to 500 bar and at a temperature of 0 to 200°C,
or, wherein the process includes the following measures:
a") provision of 4-amino-alkylpiperidine of formula VII 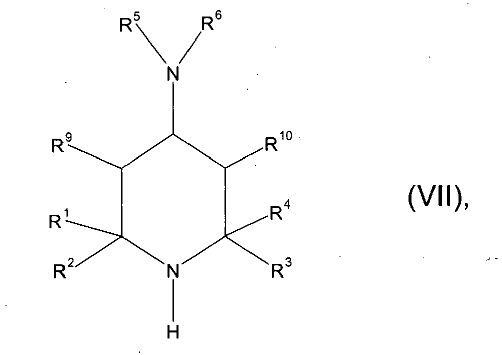 wherein R¹, R², R³, R⁴, R⁵, R⁶, R⁹ and R¹⁰ have the above-defined meaning, in one or more aprotic organic solvents
d') feeding an organic chloride of formula R⁷-Cl or of formula Cl-R⁸-(Cl)ₘ or an organic sulfate of formula R⁷-O-SO₂-O-R⁷, wherein R⁷, m and R⁸ have the above meanings, to the solution from step a"), and
e) reacting the reaction mixture from step d') at a pressure from 1 to 500 bar and at a temperature from 0 to 200°C.

2. The method according to claim 1, **characterized in that** after step e) as step f) an exchange of the chloride ion or the sulfate ion by another anion takes place, or that after step e) as step g) a separation of the reaction product from the reaction mixture takes place, or that after step e) as step f) an exchange of the chloride ion or the sulfate ion by another anion takes place and that thereafter as step g) a separation of the reaction product from the reaction mixture takes place, or that after step e) as step g) a separation of the reaction product from the reaction mixture takes place and that thereafter as step f) an exchange of the chloride ion or the sulfate ion by another anion takes place.

3. The method according to at least one of claims 1 to 2, **characterized in that** the reaction of the reaction mixture in step e) takes place in the presence of a base.

4. The method according to at least one of claims 1 to 3, **characterized in that** R¹, R², R³ and R⁴ are methyl or ethyl.

5. The method according to at least one of claims 1 to 4, **characterized in that** R⁹ and R¹⁰ are hydrogen.

6. The method according to at least one of claims 1 to 5, **characterized in that** the amine of formula HNR⁵R⁶ used in step a) is a secondary amine.

7. The method according to at least one of claims 1 to 6, **characterized in that** R⁵, R⁶ and R⁷ independently of one another are alkyl or alkyl which is optionally substituted with one or more residues selected form the group consisting of alkyl, alkoxy, hydroxy, carboxyl, carboxylic ester, carboxylic amide, sulfonate, sulfonic acid ester, sulfamide or halogen, or alkyl interrupted by one or more divalent residues selected from the group consisting of oxygen or imino, which are not directly adjacent to each other,

8. The method according to claim 7, **characterized in that** R⁵, R⁶ and R⁷ independently of one another are C₁-C₆-alkyl.

9. The method according to at least one of claims 1 to 8, **characterized in that** in step d) or d') an organic chloride is used.

10. The method according to at least one of claims 1 to 9, **characterized in that** the alcohol in steps a) to c) or a') to c) is an aliphatic alcohol with one to six carbon atoms, especially methanol or ethanol.

11. The method according to at least one of claims 1 to 10, **characterized in that** the aprotic solvent in step d) or in steps a), a') or a") is an organic nitrile and/or an aromatic hydrocarbon, preferably acetonitrile and/or toluene.

12. The method according to at least one of claims 1 to 11, **characterized in that** the method comprises steps a) to e) and g) or a) to g).

13. The method according to at least one of claims 1 to 12, **characterized in that** the method includes the manufacture of the compound of formula I.

14. A method for the manufacture of 4-ammonium-alkylpiperidin-1-yloxyl salts of formula III or of formula IV 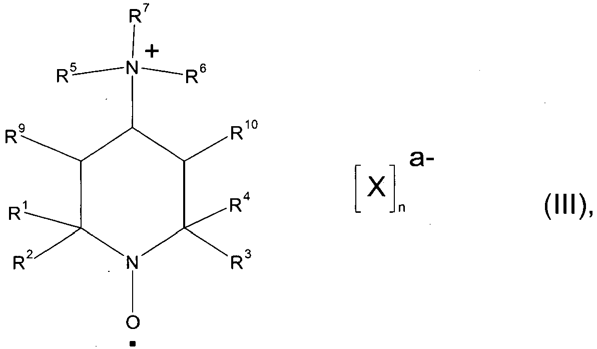 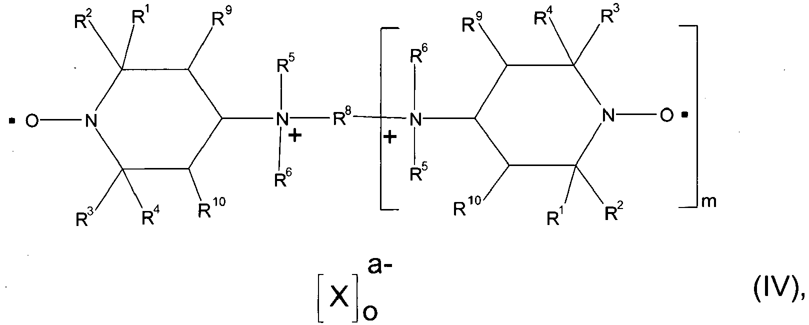
wherein R¹, R², R³ and R⁴ are C₁-C₆-alkyl,
R⁹ and R¹⁰ independently of one another are hydrogen or C₁-C₆-alkyl,
R⁵ and R⁶ independently of one another are hydrogen, alkyl, cycloalkyl, aryl, aralkyl or heterocyclyl, or alkyl, cycloalkyl, aryl, aralkyl or heterocyclyl which are substituted with one or more residues selected from the group consisting of alkyl, alkoxy, hydroxy, carboxyl, carboxylic ester, carboxylic amide, sulfonate, sulfonic acid ester, sulfonamide or halogen, or alkyl interrupted by one or more divalent residues selected from the group consisting of oxygen or imino, which are not directly adjacent to each other, or in which R⁵ and R⁶ together form an alkylene group,
R⁷ is alkyl or alkyl which is substituted with one or more residues selected from the group consisting of alkyl, alkoxy, hydroxy, nitro, carboxyl, carboxylic ester, carboxylic amide, sulfonate, sulfonic acid ester, sulfonamide or halogen, or alkyl interrupted by one or more divalent residues selected from the group consisting of oxygen or imino, which are not directly adjacent to each other,
R⁸ is a di- to sixvalent organic bridge group, or a di- to sixvalent organic bridge group, which is substituted with one or more residues selected from the group consisting of alkyl, alkoxy, hydroxy, nitro, carboxyl, carboxylic ester, carboxylic amide, sulfonate, sulfonic acid ester, sulfonamide or halogen, or an alkylene group interrupted by one or more divalent residues selected from the group consisting of oxygen or imino, which are not directly adjacent to each other,
a is an integer from 1 to 10.000,
m is an integer from 1 to 5,
n is a number with value 1/a,
o is a number with value (1 + m) / a, and
X is an organic anion, an inorganic anion or a mixture of these anions, wherein the process includes the following measures:
execution of the process with steps a) to e) and optionally f) and /or g) or with steps a') and b) to e) and optionally f) and /or g) or with steps a"), d') and e) and optionally f) and/or g) according to any of claims 1 to 13, and
h) reacting the reaction product from step e), f) or g) according to one of claims 1 to 13 with a peroxide.

15. The method according to claim 14, **characterized in that** the peroxide is hydrogen peroxide.

16. The method according to at least one of claims 14 to 15, **characterized in that** in step h) the reaction product from step e) is used directly.

17. The method according to at least one of claims 14 to 16, **characterized in that** to the reaction product from step e) alkali hydroxide and sodium tungstate are added followed by treatment with hydrogen peroxide or that to the reaction product from step e) earth alkaline sulfate is added followed by treatment with hydrogen peroxide.

## Revendications

1. Procédé de préparation de sels de 4-ammonium-2,2,6,6-tétraalkylpipéridine de formule I ou de formule II 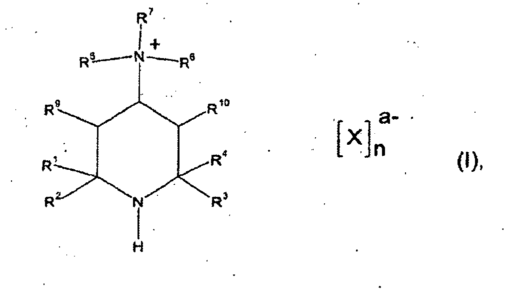 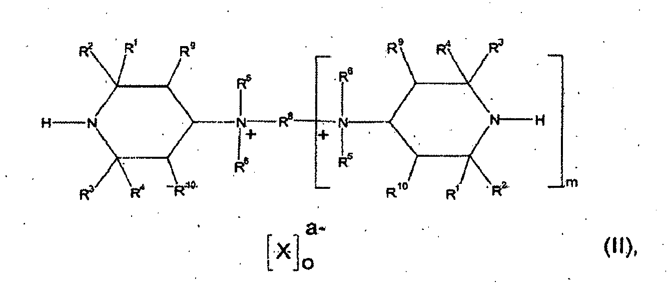
dans lesquelles R¹, R², R³ et R⁴ sont un alkyle en C₁ à C₆,
R⁹ et R¹⁰ représentent, indépendamment l'un de l'autre, de l'hydrogène ou un alkyle en C₁ à C₆,
R⁵ et R⁶ représentent, indépendamment l'un de l'autre, de l'hydrogène, un alkyle, un cycloalkyle, un aryle, un aralkyle ou un hétérocyclyle, ou un alkyle, un cycloalkyle, un aryle, un aralkyle ou un hétérocyclyle qui est substitué par un ou plusieurs radicaux choisis dans le groupe constitué par un alkyle, un alcoxyle, un hydroxyle, un carboxyle, un ester carboxylique, un carboxylamide, un sulfonate, un ester d'acide sulfonique, un sulfonamide ou un halogène, ou un alkyle qui est interrompu par un ou plusieurs radicaux bivalents non directement adjacents les uns aux autres, choisis dans le groupe constitué par l'oxygène ou un imino, ou dans lesquelles R⁵ et R⁶ forment ensemble un groupe alkylène,
R⁷ représente un alkyle ou un alkyle qui est substitué par un ou plusieurs radicaux choisis dans le groupe constitué par un alkyle, un alcoxyle, un hydroxyle, un nitro, un carboxyle, un ester carboxylique, un carboxylamide, un sulfonate, un ester sulfonique, un sulfonamide ou un halogène, ou un alkyle qui est interrompu par un ou plusieurs radicaux bivalents non directement adjacents les uns aux autres, choisis dans le groupe constitué par l'oxygène ou un imino,
R⁸ est un groupe de pontage organique bivalent à hexavalent, ou un groupe de pontage organique bivalent à hexavalent qui est substitué par un ou plusieurs radicaux choisis dans le groupe constitué par un alkyle, un alcoxyle, un hydroxyle, un nitro, un carboxyle, un ester carboxylique, un carboxylamide, un sulfonate, un ester sulfonique, un sulfonamide ou un halogène, ou un groupe alkylène qui est interrompu par un ou plusieurs radicaux bivalents non directement adjacents les uns aux autres, choisis dans le groupe constitué par l'oxygène ou un imino,
a représente un nombre entier de 1 à 10 000,
m représente un nombre entier de 1 à 5,
n représente un nombre ayant la valeur 1 /a,
o représente un nombre ayant la valeur (1 + m) / a, et
X est un anion organique, un anion inorganique ou un mélange de ces anions, le procédé comprenant les mesures suivantes :
a) mise à disposition d'une solution de 4-oxo-alkylpipéridine de formule VI 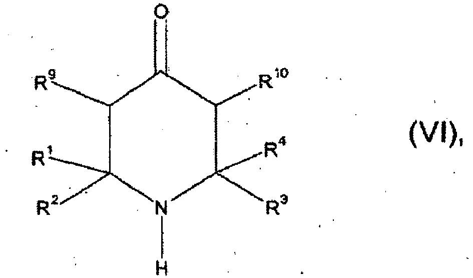 dans laquelle R¹, R², R³, R⁴, R⁹ et R¹⁰ sont tels que définis ci-dessus, et d'une amine de formule HNR⁵R⁶, dans laquelle R⁵ et R⁶ sont tels que définis ci-dessus, dans un solvant choisi dans le groupe constitué par les alcools, les éthers, les nitriles, les hydrocarbures halogénés, les hydrocarbures aromatiques, les hydrocarbures aliphatiques ou leurs mélanges,
b) amination réductrice du composé 4-oxo-alkylpipéridinyle de formule VI au moyen d'hydrogène, de métaux alcalins, de magnésium, d'aluminium, de zinc, d'aluminohydrures alcalins, de borohydrures alcalins, d'hydrures de métaux alcalins, d'hydrures de métaux alcalino-terreux, de sulfites de métaux alcalins, de dithionites de métaux alcalins, de thiosulfates de métaux alcalins ou d'hydrazine. en présence d'un catalyseur d'hydrogénation, pour obtenir un composé 4-amino-alkylpipéridinyle de formule VII 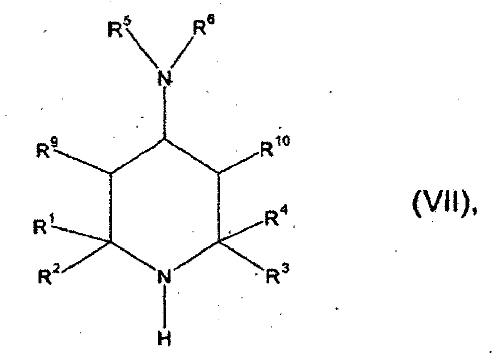 dans laquelle R¹, R², R³, R⁴, R⁵, R⁶, R⁹ et R¹⁰ sont tels que définis ci-dessus,
c) séparation du produit de réaction de formule VII issu de l'étape b) du catalyseur d'hydrogénation et du solvant,
d) dissolution du produit de réaction de formule VII séparé à l'étape c) dans un ou plusieurs solvants organiques aprotiques et ajout d'un chlorure organique de formule R⁷ à Cl ou de formule Cl-R⁸-(Cl)ₘ ou d'un sulfate organique de formule R⁷-O-SO₂-O-R⁷, dans laquelle R⁷, m et R⁸ sont tels que définis ci-dessus, et
e) mise à réagir du mélange réactionnel issu de l'étape d) à une pression de 1 à 500 bars et à une température de 0 à 200 °C,
ou le procédé comprenant les mesures suivantes :
a') mise à disposition d'une solution d'une imine de formule V 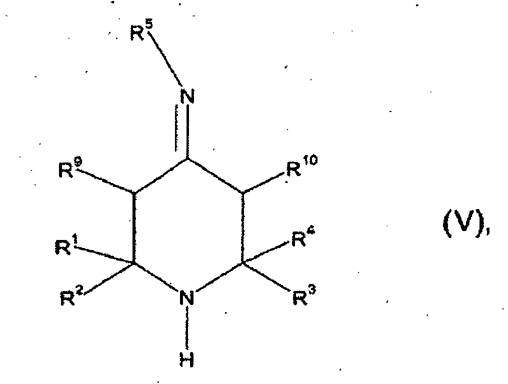 dans laquelle R₁, R², R³, R⁴, R⁵, R⁹ et R¹⁰ sont tels que définis ci-dessus, dans un solvant choisi dans le groupe constitué par les alcools, les éthers, les nitriles, les hydrocarbures halogénés, les hydrocarbures aromatiques, les hydrocarbures aliphatiques ou leurs mélanges,
b) hydrogénation de l'imine de formule V au moyen d'hydrogène, de métaux alcalins, de magnésium, d'aluminium, de zinc, d'aluminohydrures alcalins, de borohydrures alcalins, d'hydrures de métaux alcalins, d'hydrures de métaux alcalino-terreux, de sulfites de métaux alcalins, de dithionites de métaux alcalins, de thiosulfates de métaux alcalins ou d'hydrazine. en présence d'un catalyseur d'hydrogénation, pour obtenir un composé 4-amino-alkylpipéridinyle de formule VII 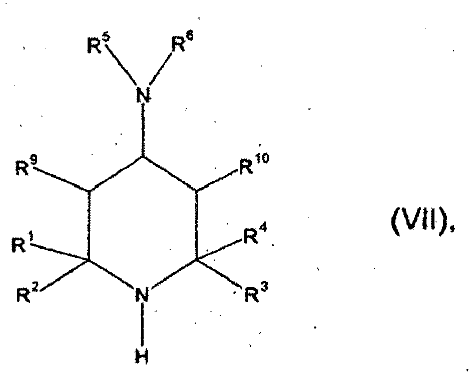 dans laquelle R¹, R², R³, R⁴, R⁵, R⁶, R⁹ et R¹⁰ sont tels que définis ci-dessus,
c) séparation du produit de réaction de formule VII issu de l'étape b) du catalyseur d'hydrogénation et du solvant,
d) dissolution du produit de réaction de formule VII séparé à l'étape c) dans un ou plusieurs solvants organiques aprotiques et ajout d'un chlorure organique de formule R⁷ à Cl ou de formule Cl-R⁸-(Cl)ₘ ou d'un sulfate organique de formule R⁷-O-SO₂-O-R⁷, dans laquelle R⁷, m et R⁸ sont tels que définis ci-dessus, et
e) mise à réagir du mélange réactionnel issu de l'étape d) à une pression de 1 à 500 bars et à une température de 0 à 200 °C,
ou le procédé comprenant les mesures suivantes :
a") mise à disposition de 4-amino-alkylpipéridine de formule VII 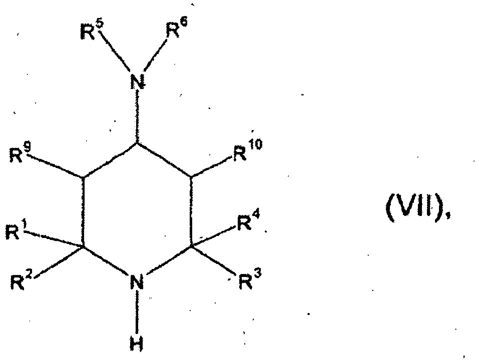 dans laquelle R¹, R², R³, R⁴, R⁵, R⁶, R⁹ et R¹⁰ sont tels que définis ci-dessus, dans un ou plusieurs solvants organiques aprotiques,
d') ajout d'un chlorure organique de formule R⁷ à Cl ou de formule Cl-R⁸-(Cl)ₘ ou d'un sulfate organique de formule R⁷-O-SO₂-O-R⁷, dans laquelle R⁷, m et R⁸ sont tels que définis ci-dessus, à la solution de l'étape a"), et
e) mise à réagir du mélange réactionnel issu de l'étape d') à une pression de 1 à 500 bars et à une température de 0 à 200 °C.

2. Procédé selon la revendication 1, **caractérisé en ce qu'**après l'étape e), on effectue, en tant qu'étape f), un échange de l'ion chlorure ou de l'ion sulfate par un autre anion, ou qu'après l'étape e), on effectue, en tant qu'étape g), une séparation du produit de réaction du mélange réactionnel, ou qu'après l'étape e), on effectue, en tant qu'étape f), un échange de l'ion chlorure ou de l'ion sulfate par un autre anion et qu'on effectue ensuite, en tant qu'étape g), une séparation du produit de réaction du mélange réactionnel, ou qu'après l'étape e), on effectue, en tant qu'étape g), une séparation du produit de réaction du mélange réactionnel et qu'on effectue ensuite, en tant qu'étape f), un échange de l'ion chlorure ou de l'ion sulfate par un autre anion.

3. Procédé selon au moins l'une des revendications 1 à 2, **caractérisé en ce que** le mélange réactionnel de l'étape e) est mis à réagir en présence d'une base.

4. Procédé selon au moins l'une des revendications 1 à 3, **caractérisé en ce que** R₁, R², R³ et R⁴ sont des groupes méthyle ou éthyle.

5. Procédé selon au moins l'une des revendications 1 à 4, **caractérisé en ce que** R⁹ et R¹⁰ sont de l'hydrogène.

6. Procédé selon au moins l'une des revendications 1 à 5, **caractérisé en ce que** l'amine de formule HNR⁵R⁶ utilisée à l'étape a) est une amine secondaire.

7. Procédé selon au moins l'une des revendications 1 à 6, **caractérisé en ce que** R⁵, R⁶ et R⁷ représentent, indépendamment les uns des autres, un alkyle ou un alkyle éventuellement substitué par un ou plusieurs radicaux choisis dans le groupe constitué par un alkyle, un alcoxyle, un hydroxyle, un carboxyle, un ester carboxylique, un carboxylamide, un sulfonate, un ester sulfonique, un sulfamide ou un halogène, ou un alkyle interrompu par un ou plusieurs radicaux bivalents non directement adjacents les uns aux autres, choisis dans le groupe constitué par l'oxygène ou un imino.

8. Procédé selon la revendication 7, **caractérisé en ce que** R⁵, R⁶ et R⁷ représentent, indépendamment les uns des autres, un alkyle en C₁ à C₆.

9. Procédé selon au moins l'une des revendications 1 à 8, **caractérisé en ce qu'**on utilise un chlorure organique à l'étape d) ou d').

10. Procédé selon au moins l'une des revendications 1 à 9, **caractérisé en ce que** l'alcool des étapes a) à c) ou a') à c) est un alcool aliphatique ayant un à six atomes de carbone, en particulier le méthanol ou l'éthanol.

11. Procédé selon au moins l'une des revendications 1 à 10, **caractérisé en ce que** le solvant aprotique de l'étape d) ou des étapes a), a') ou a") est un nitrile organique et/ou un hydrocarbure aromatique, en particulier l'acétonitrile et/ou le toluène.

12. Procédé selon au moins l'une des revendications 1 à 11, **caractérisé en ce que** le procédé comprend les étapes a) à e) et g) ou a) à g).

13. Procédé selon au moins l'une des revendications 1 à 12, **caractérisé en ce que** le procédé comprend la préparation du composé de formule I.

14. Procédé de préparation de sels de 4-ammonium-alkylpipéridine-1-yloxyl de formule III ou de formule IV 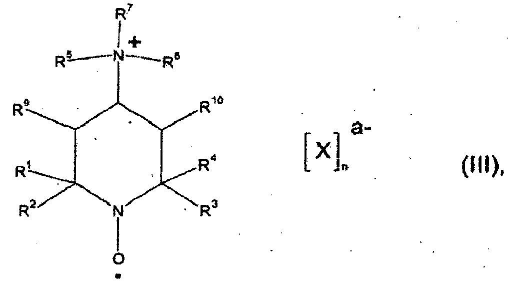 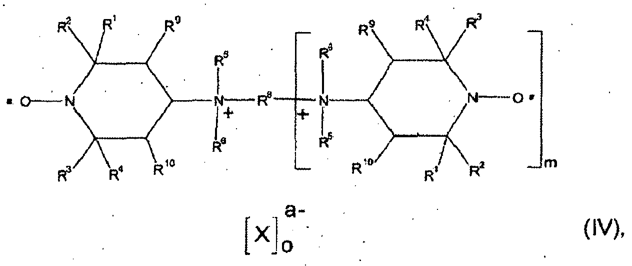
dans lesquelles R¹, R², R³ et R⁴ sont un alkyle en C₁ à C₆,
R⁹ et R¹⁰ représentent, indépendamment l'un de l'autre, de l'hydrogène ou un alkyle en C₁ à C₆,
R⁵ et R⁶ représentent, indépendamment l'un de l'autre, de l'hydrogène, un alkyle, un cycloalkyle, un aryle, un aralkyle ou un hétérocyclyle, ou un alkyle, un cycloalkyle, un aryle, un aralkyle ou un hétérocyclyle qui est substitué par un ou plusieurs radicaux choisis dans le groupe constitué par un alkyle, un alcoxyle, un hydroxyle, un carboxyle, un ester carboxylique, un carboxylamide, un sulfonate, un ester d'acide sulfonique, un sulfonamide ou un halogène, ou un alkyle qui est interrompu par un ou plusieurs radicaux bivalents non directement adjacents les uns aux autres, choisis dans le groupe constitué par l'oxygène ou un imino, ou dans lesquelles R⁵ et R⁶ forment ensemble un groupe alkylène,
R⁷ représente un alkyle ou un alkyle qui est substitué par un ou plusieurs radicaux choisis dans le groupe constitué par un alkyle, un alcoxyle, un hydroxyle, un nitro, un carboxyle, un ester carboxylique, un carboxylamide, un sulfonate, un ester sulfonique, un sulfonamide ou un halogène, ou un alkyle qui est interrompu par un ou plusieurs radicaux bivalents non directement adjacents les uns aux autres, choisis dans le groupe constitué par l'oxygène ou un imino,
R⁸ est un groupe de pontage organique bivalent à hexavalent, ou un groupe de pontage organique bivalent à hexavalent qui est substitué par un ou plusieurs radicaux choisis dans le groupe constitué par un alkyle, un alcoxyle, un hydroxyle, un nitro, un carboxyle, un ester carboxylique, un carboxylamide, un sulfonate, un ester sulfonique, un sulfonamide ou un halogène, ou un alkyle qui est interrompu par un ou plusieurs radicaux bivalents non directement adjacents les uns aux autres, choisis dans le groupe constitué par l'oxygène ou un imino,
a représente un nombre entier de 1 à 10 000,
m représente un nombre entier de 1 à 5,
n représente un nombre ayant la valeur 1 /a,
o représente un nombre ayant la valeur (1 + m) / a, et
X est un anion organique, un anion inorganique ou un mélange de ces anions,
le procédé comprenant les mesures suivantes :
exécution du procédé avec les étapes a) à e) et éventuellement f) et/ou g) ou avec les étapes a') et b) à e) et éventuellement f) et/ou g) ou avec les étapes a"), d') et e) et éventuellement f) et/ou g) selon l'une des revendications 1 à 13, et
h) mise à réagir du produit de réaction issu de l'étape e), f) ou g) selon l'une des revendications 1 à 13 avec un peroxyde.

15. Procédé selon la revendication 14, **caractérisé en ce que** le peroxyde est du peroxyde d'hydrogène.

16. Procédé selon au moins l'une des revendications 14 ou 15, **caractérisé en ce que** le produit de réaction issu de l'étape e) est utilisé directement à l'étape h).

17. Procédé selon au moins l'une des revendications 14 à 16, **caractérisé en ce que** le produit de réaction issu de l'étape e) est mélangé avec de l'hydroxyde de métal alcalin et du tungstate de sodium et ensuite traité avec du peroxyde d'hydrogène, ou que le produit de réaction issu de l'étape e) est mélangé avec du sulfate de métal alcalino-terreux et ensuite traité avec du peroxyde d'hydrogène.
